# EUROPEAN PATENT APPLICATION

(11) **EP 2 361 611 A2**
(43) Date of publication of application: **31.08.2011**
(21) Application number: 10015416.0
(22) Date of filing: 09.06.2005
(51) Int. Cl.: A61K 9/10, A61K 9/16, A61K 47/48, A61K 31/135

(54) **Aqueous sustained-release drug delivery system for highly water-soluble electrolytic drugs**

(62) Divisional of application: 05758570.5
(71) Applicant: Upm Pharmaceuticals, Inc., Baltimore, MD 21224 (US)
(72) Inventor: Hollenbeck, R., Gary, Ellicott City, MD 21043 (US)
(74) Representative: Soames, Candida Jane

(57) **Abstract**

A liquid form controlled release drug composition, wherein said composition is capable of controlled release of the drug over at least 8 hours, comprising:
a. a dispersed phase comprising an ion-exchange matrix drug complex comprising a pharmaceutically acceptable ion-exchange matrix and a water-soluble electrolytic drug associated with the ion-exchange matrix, wherein the surface charge of the ion-exchange matrix is opposite that of the electrolytic drug; and
b. a dispersion medium comprising 50% to 70% on a weight by weight basis of a highly hydrated excipient.

## Description

This application is a continuation-in-part of U.S. Application No. 10/724,276, filed November 26, 2003, which is entitled to and claims priority benefit under 35 U.S.C. §119(e) to U.S. Provisional Application No. 60/429,202, filed November 26, 2002, each of which is incorporated herein by reference in its entirety.

### 1. FIELD OF THE INVENTION

The present invention relates to liquid sustained release suspension dosage forms comprising ionized forms of water-soluble drugs.

### 2. BACKGROUND OF THE INVENTION

Relative to solid oral dosage forms, liquid formulations have the distinct advantages of dosage flexibility and ease of swallowing. In addition, it is possible to administer, in a single volume of liquid, a relatively large quantity of dispersed solid, which would normally require several tablets or capsules. Moreover, there is a recognized need for sustained release formulations to be available in a convenient, easy-to-take liquid dosage form. However, the formulation of liquid oral suspensions having sustained-released capabilities has only resulted in limited success. In fact, liquid oral suspensions having a 24 hour sustained release profile has been extremely difficult to achieve. In part, this is due to the challenges presented in maintaining the stability of sustained-release particles when present in liquid dispersal systems, the difficulty in achieving sustained release of the drug from the dispersed phase, as well as the problems encountered when attempting to achieve a low free drug concentration in the dispersion medium.

Traditionally, the attempts to achieve a liquid oral dosage formulation capable of sustained release have focused on controlling the release of drug from the dispersed phase of a liquid dosage form. One method for controlling the release rate of the drug involves dispersing the drug into a liquid medium to form a drug-dispersed phase and a continuous phase. Simple, traditional pharmaceutical suspensions are liquid dosage forms consisting of a dispersed drug phase that has low solubility in a dispersion medium. Such systems, whether administered orally or parenterally, have a low concentration of drug in the continuous phase and offer an inherent sustained-release of drug, controlled at least in part, by the rate of the dissolution process itself.

Fundamental ion-exchange technology has been an approach utilized for achieving sustained release for solid dosage forms and various attempts have been made to further utilize the technology in liquid suspension formulations as well. For example, U.S. Patent No. 2,990,332 to Keating and Y. Raghunathan et al., J. Pharm. Sci.. 70: 379-84 (1981) disclose a method of controlling the release rate of drug by adsorbing the salt form of a drug onto a carrier resin such as an ion-exchange resin. However, the release rate of the adsorbed drug can only be controlled by the rate at which it is displaced by counter ions. T. Tarvainen et al., Biomaterials 20:2177-83 (1999) discloses a formulation wherein cationic and neutral forms of drugs are adsorbed onto a porous poly(vinylidene fluoride)-poly(acrylic acid) membrane. According to this reference, the release rate of the cationic drugs is affected by the ionic strength of the dissolution media. U.S. Patent No. 6,247,174 B 1 to Hom-ma et al. discloses solid oral dosage forms wherein the ion-exchange resin calcium-alginate is a carrier for delivery of a medicament which is only slightly soluble in water.

However, a recurring problem with traditional ion-exchange resin drug complexes is the rapid rate of counterion exchange, which causes the drug to be released too rapidly or in an uncontrolled manner from the ion-exchange resin.

Diffusion through a porous membrane is one of the classical approaches to achieve sustained release for solid dosage forms, and the Pennkinetic system utilizes a membrane coat to provide diffusion control for the traditional ion-exchange resin drug complex. For example, U.S. Patent No. 4,996,047 to Kelleher et al discloses drug-ion-exchange resin complex particles coated with a water-impermeable diffusion barrier layer. S. Motycka et al., J. Pharm. Sci. 74:643-46 (1985) and M.G. Moldenhauyer et al., J. Pharm. Sci., 79:659-66 (1990) disclose a suspension of particles comprising a complex formed between an anionic resin and theophylline, where the particles are coated with ethylcellulose, paraffin, or both, and the coating controls the release rate of the drug. According to U.S. Patent No. 5,376,384 to Eichel et al.*,* a drug formulation wherein a water soluble drug core is coated with a hydratable diffusion barrier allegedly provides prolonged, delayed and sustained delivery of the drug from a solid dosage form.

However, even though the Pennkinetic ion-exchange system was introduced over 20 years ago, only one product utilizing this technology exists in the market place, possibly due to: the poor suitability of the ion-exchange resin (*i.e.,* hydrophobicity and swelling); complex formulation and manufacturing processes are required; and long term stability problems. The traditional approach taken to circumvent the problems associated with ion-exchange technology and the application of diffusion membranes or coatings in preparing liquid oral dosage forms is the use of various impregnating or solvating agents.

For example, U.S. Patent No. 4,859,461 to Chow et al. discloses a drug-ion-exchange resin formulation wherein sulfonic acid cationic exchange resin particles and an impregnating agent are allegedly useful for improving the coatablility of the particles. U.S. Patent No. No. 4,221,778 to Raghunathan discloses an extended release pharmaceutical preparation wherein an impregnating or solvating agent is added to retard the swelling of ion-exchange drug resin complex particles, and a water-permeable diffusion barrier coating allegedly delays the release rate of the drug (*see also* EP 171,528; EP 254,811, and EP 254,822). U.S. Patent No. 5,186,930 to Kogan et al. discloses multi-coated drug-resin particles with an inner wax coating and an outer diffusion-controlling polymer coating. According to the patent, the wax coating is allegedly useful for preventing leaching of drug through the polymer coating as well as drug dumping caused by swelling of the resin and subsequent cracking of the polymer coating. U.S. Patent No. 4,762,709 to Scheumaker discloses a formulation wherein a coated first drug-ion-exchange resin particle is suspended in a liquid carrier with an uncoated second drug-ion-exchange resin component bearing the same charge as the first drug in the coated first drug-ion-exchange resin particle. According to the reference, the release rate of the first drug from the coated first drug-ion-exchange resin particle is increased when the second drug is present in the second uncoated drug-ion-exchange resin complex compared to when the second drug is included with the first drug in the coated first drug-ion-exchange resin. A product based on the this ion-exchange technology is Tussionex® (Hydrocodone Polistirex and Chlorpheniramine Polistirex) Pennkinetic® Extended Release Suspension (Celltech Pharmaceuticals, Inc.), which was recently approved by the U.S. Food and Drug Administration.

There also remain challenges in maintaining the physical stability of controlled release suspension dosage forms consisting of coated beads dispersed in an aqueous vehicle. Specifically, because the core bead is "dry", and the components hydrophilic, diffusion of water across the coating and into the bead produces significant swelling and the development of high osmotic pressure "inside" the bead. This swelling can then lead to the disruption of coatings and certain failure of any control release mechanism reliant on bead coatings.

There remains a need for sustained release liquid dosage forms, in particular dosage forms with better pharmacologic properties and stability and dosage forms which will appeal to the commercial marketplace. It remains a challenge to achieve pharmaceutically acceptable suspension liquid dosage forms containing the active ingredient in the dispersed phase, having a low free drug concentration in the dispersion medium, and capable of providing sustained drug release from the dispersed phase after administration to a patient. In particular, there remains a need for sustained release liquid dosage forms, suitable for once-a-day or twice-a-day administration of highly water soluble electrolytic drugs.

Citation of any reference in Section 2 of this application is not an admission that the reference is prior art to the application.

### 3. SUMMARY OF THE INVENTION

The present invention is based in part on the discovery that a stable liquid sustained release suspension dosage form with low free drug concentration in the dispersion medium can be achieved by confining a water soluble electrolytic drug in the dispersed phase of a suspension by controlling osmotic pressure and by utilizing thermodynamic electrostatic interactions.

The inventors have overcome the challenges presented by a drug delivery system that comprises beads containing drug that are coated with a material capable of controlling release of the highly soluble drug and immersed in an aqueous dispersion medium. In particular, the inventors have demonstrated that by manipulating the components of the bead and selection of the suspension medium, initial water penetration into the bead can be precluded and the drug can remain sequestered in the dispersed phase until administration to a patient. Moreover, the inventors have demonstrated that by controlling shifts in the osmotic pressure, the traditional challenges of keeping coatings intact are overcome and controlled release can be readily achieved using polymeric porous or non-porous membranes. In addition, products utilizing the drug delivery systems of the invention have a long shelf life since the drug remains confined in the dispersed phase and any functional coatings remain intact.

As such, the instant invention encompasses liquid sustained release suspension dosage forms that have the following characteristics: capability for maintaining the physical integrity of the coating both *in vitro (e.g.,* in the bottle or in storage) and *in vivo* for a period long enough to control release; control of water activity such that the water present outside the beads in the product is at an activity less than inside the bead; and the presence of free dissolved drug exists in the reservoir available to diffuse out once the drug is administered. In particular, the invention contemplates the inclusion of a soluble, non-electrolytic component(s) within the bead during manufacture. Such a component will dissolve when water is absorbed into the bead and diffuse out of the reservoir and reduce osmotic pressure, thereby reducing swelling of the bead and loss of integrity of the bead coating. Accordingly, the invention encompasses the use of excipients that are capable of being mass transferred out of the drug-loaded bead when the bead absorbs water, thereby reducing pressure inside the bead and preventing the disruption of bead coatings and facilitating controlled release of the drug.

The inventors have also made the surprising discovery that adding a high concentration of an inactive highly hydrated excipient to the dispersion medium can impede dissolution of the drug in the dispersed phase, and that free drug concentration in the dispersion medium is extremely low, virtually non-existent. As such, the invention also contemplates reducing the water activity in the dispersion medium by adding a high concentration of inactive component that is highly hydrated and capable of associating strongly with water and impeding the association of water with the drug complex of the dispersed phase. As such, water in the dispersion medium is not sufficiently attracted to the drug loaded bead, thereby eliminating dissolution of drug prior to administration and confining the drug in the dispersed phase of a suspension.

The inventors have also demonstrated that a drug can be confined in a dispersed phase of a suspension by virtue of it being a counter-ion in the diffuse double layer of a hydrophilic colloid and that the drug distribution in the dispersed phase and the dispersion medium can be manipulated by the presence of similarly charged (vis-à-vis the drug) non-diffusible polyelectrolytes in the dispersion medium. The result is an effective and thermodynamically stable drug "binding" mechanism capable of circumventing the challenges presented in traditional approaches (for example, coating a drug product), which attempt to mechanically "hold back" the natural tendency of the active ingredient to move from a region of high concentration to lower concentration.

Accordingly, the present invention encompasses thermodynamically stable liquid dosage drug suspensions possessing a low free drug concentration in the dispersion medium, capable of providing sustained drug release when administered to a patient. The present invention encompasses a novel class of sustained release drug formulations comprising a drug confined in a dispersed phase of a suspension by a pharmaceutically acceptable ion-exchange matrix having a surface charge opposite that of the drug. Such liquid formulations are capable of achieving sustained release over 24 hours, up to 48 hours. As such, the invention encompasses liquid dosage forms that need only be administered once daily, ensuring ease of administration. In certain embodiments, the dispersion medium is substantially free of diffusible counterions capable of displacing the drug from the ion exchange matrix. It is envisaged by the invention that a soluble non-electrolytic component, having relatively low molecular weight may also be included in the drug complex. Preferably the drug-ion exchange matrix-complex is a bead. It is also envisaged by the invention that the dispersed phase can optionally be membrane-coated with a porous or non-porous polymeric membrane. It is further envisaged that the dispersion medium can optionally include a polyelectrolyte with the same charge as the drug. The inclusion of a like-charged, polyelectrolyte in the dispersion medium, which is not capable of diffusing through the membrane, further forces the lower molecular weight drug into the dispersed phase (through Donnan membrane effect) where it is already confined by electrostatic force. Drug "release" is triggered when the suspension is placed in an environment, for example gastric or intestinal fluid, with high concentrations of water and small ions that possess the same charge as the drug, as the small ions swamp the diffuse double layer. In alternate embodiments, the dispersion medium also includes a highly hydrated excipient(s) capable of associating closely with water, thereby limiting the water activity in the dispersion medium and impeding the dissolution of drug prior to administration.

As such, in one embodiment of the invention, the drug is confined in the dispersed phase of a suspension through thermodynamically stable electrostatic interactions. Optionally, the dispersed phase is membrane-coated and in an alternate embodiment, the drug is confined in the dispersed phase of a suspension through Donnan membrane effects. In preferred embodiments, the drug is confined in the dispersed phase of a suspension through thermodynamically stable electrostatic interactions and Donnan membrane effects. In other preferred embodiments, the drug is confined in the dispersed phase of a suspension by a highly hydrated component in the dispersion medium which strongly associates with water in the dispersion medium. As a result, water is less attracted to the components of the drug loaded bead. In all of the embodiments, drug release is activated following administration to a patient. In various embodiments, sustained release is achieved when the patient's body conditions disrupt the osmotic effects, electrostatic interactions and/or the Donnan membrane effects. In a particular embodiment, sustained release is achieved when the patient's body allows the drug to diffuse through the ion-exchange matrix and/or the membrane. In certain embodiments, the gastric fluid of the patient dilutes the dispersion medium after administration of the dosage form and the membrane becomes hydrated and more porous, allowing dissolution and dispersion of drug from the beads.

The present invention contemplates liquid form controlled release compositions wherein the amount of free drug in the dispersion medium is less than 10%, preferably less than 5%, more preferably less than 0.5%, most preferably less than 0.05% based on the total molar amount of drug in the dispersion medium and dispersed phase. In such embodiments, the dispersion medium is substantially devoid of free drug and the sustained release formulation is physically and chemically stable for more than one year, preferably more than about 2 years, preferably more than about 3 years, most preferably more than 4 years.

In one embodiment, the present invention relates to a liquid form controlled release drug composition, comprising:
(a) a dispersed phase comprising a water-soluble electrolytic drug associated with a pharmaceutically acceptable ion-exchange matrix, wherein the surface charge of the ion-exchange matrix is opposite that of the electrolytic drug; and
(b) a dispersion medium substantially free of diffusible counterions.

In another embodiment, the present invention relates to a liquid form controlled release drug composition, comprising:
(a) a dispersed phase comprising an ion-exchange matrix drug complex comprising a pharmaceutically acceptable ion-exchange matrix and a water-soluble electrolytic drug associated with the ion-exchange matrix, wherein the surface charge of the ion-exchange matrix is opposite that of the electrolytic drug; and
(b) a dispersion medium substantially free of diffusible counterions.

In another embodiment, the dispersion medium further comprises polyelectrolytes having the same charge as the electrolytic drug.

In another embodiment, the dispersion medium further comprises a highly hydrated excipient that attracts water in the dispersion medium.

In another embodiment, the dispersed phase is membrane-coated. In particular embodiments, the membrane is polymeric. The membrane can be porous or non-porous. In a particular preferred embodiment, the membrane controls diffusion of the drug.

In another embodiment, the interaction between the ion-exchange matrix and drug controls release of the drug and/or the amount of free drug in the dispersion medium.

In yet another embodiment, the dispersion medium comprises a polyelectrolyte having the same charge as the electrolytic drug and the dispersed phase is membrane-coated, wherein diffusion of the drug and any swelling of the ion-exchange drug complex is controlled by Donnan membrane effects.

In yet other embodiments, the dispersed phase comprises drug-loaded beads that include a low molecular weight, non-electrolytic soluble excipient(s) capable of dissolving and diffusing out of the beads when water is absorbed into the bead and reducing osmotic pressure inside the beads.

In one embodiment, the present invention relates to a liquid form controlled release drug composition, comprising:
(a) a dispersed phase comprising a water-soluble electrolytic drug associated with a pharmaceutically acceptable ion-exchange matrix, wherein the surface charge of the ion-exchange matrix is opposite that of the electrolytic drug; and
(b) a dispersion medium comprising a polyelectrolyte having the same charge as the electrolytic drug.

In another embodiment, the present invention relates to a liquid form controlled release drug composition, comprising:
(a) a dispersed phase comprising an ion-exchange matrix drug complex comprising a pharmaceutically acceptable ion-exchange matrix and a water-soluble electrolytic drug associated with the ion-exchange matrix, wherein the surface charge of the ion-exchange matrix is opposite that of the electrolytic drug; and
(b) a dispersion medium comprising a polyelectrolyte having the same charge as the electrolytic drug.

In another embodiment, the dispersion medium further comprises a highly hydrated excipient that attracts water in the dispersion medium.

In another embodiment, the dispersed phase is membrane-coated. In particular embodiments, the membrane is polymeric and can be porous or non-porous. In a particular preferred embodiment, the membrane controls diffusion of the drug.

In another embodiment, the interaction between the ion-exchange matrix and drug controls release of the drug and/or the amount of free drug in the dispersion medium.

In yet another embodiment, the dispersion medium comprises a polyelectrolyte having the same charge as the electrolytic drug and the dispersed phase is membrane-coated, wherein diffusion of the drug and any swelling of the ion-exchange drug complex is controlled by Donnan membrane effects.

The invention further contemplates that the dispersion medium of the above embodiment is substantially free of diffusible counterions.

The present invention also relates to methods for making the liquid form controlled release drug composition. In one embodiment, the invention relates to methods for preparing a liquid form controlled release drug composition, comprising:
(a) allowing a water-soluble electrolytic drug to associate with an ion-exchange matrix to form an ion-exchange matrix drug complex; and
(b) dispersing the ion-exchange matrix drug complex into a dispersion media substantially free of diffusible counterions; wherein
   the surface of the ion-exchange matrix has a charge opposite that of the electrolytic drug.

In another embodiment, the invention relates to methods for preparing a liquid form controlled release drug composition, comprising:
(a) allowing a water-soluble electrolytic drug to associate with an ion-exchange matrix to form an ion-exchange matrix drug complex; and
(b) dispersing the ion-exchange matrix drug complex into a dispersion media substantially free of diffusible counterions; wherein
   the surface of the ion-exchange matrix has a charge opposite that of the electrolytic drug; and
   the polyelectrolyte has the same charge as that of the electrolytic drug.

In another embodiment, the invention relates to methods for preparing a liquid form controlled release drug composition, comprising:
(a) allowing the acid form of an acid-functional ion-exchange matrix to associate with the base form of an amine-based drug to form an ion-exchange matrix drug complex; and
(b) dispersing the ion-exchange matrix drug complex into a dispersion media substantially free of diffusible counterions.

In another embodiment, the invention relates to methods for preparing a liquid form controlled release drug composition, comprising:
(a) allowing the base form of an amine-functional ion-exchange matrix to associate with the acid form of acid-based drug to form an ion-exchange matrix drug complex; and
(b) dispersing the ion-exchange matrix drug complex into a dispersion media substantially free of diffusible counterions.

In another embodiment, the invention relates to methods for preparing a liquid form controlled release drug composition, comprising:
(a) allowing a water-soluble electrolytic drug to associate with an ion-exchange matrix to form an ion-exchange matrix drug complex; and
(b) dispersing the ion-exchange matrix drug complex into a dispersion media comprising a polyelectrolyte; wherein
   the surface of the ion-exchange matrix has a charge opposite that of the electrolytic drug; and
   the polyelectrolyte has the same charge as that of the electrolytic drug.

In another embodiment, the invention relates to methods for preparing a liquid form controlled release drug composition, comprising:
(a) allowing the acid form of an acid-functional ion-exchange matrix to associate with the base form of an amine-based drug to form an ion-exchange matrix drug complex; and
(b) dispersing the ion-exchange matrix drug complex into a dispersion media comprising a polyelectrolyte; wherein
   the polyelectrolyte has a positive charge.

In another embodiment, the invention relates to methods for preparing a liquid form controlled release drug composition, comprising:
(a) allowing the base form of an amine-functional ion-exchange matrix to associate with the acid form of acid-based drug to form an ion-exchange matrix drug complex; and
(b) dispersing the ion-exchange matrix drug complex into a dispersion media comprising a polyelectrolyte; wherein
   the polyelectrolyte has a negative charge.

In all of the above embodiments, the invention also further contemplates dosage forms and methods that encompass the formation of uncomplicated aqueous gels comprising drug and ion-exchange matrix. Preferably, such aqueous gels are substantially free of spectator ions, and are spontaneously formed by reacting and hydrating a drug and an ion-exchange matrix having a charge opposite that of the drug. In certain embodiments, manipulating drug and ion-exchange matrix in solid forms having low to limited aqueous solubility can allow formation of a stable colloidal dispersion which ultimately allows a highly water soluble drug to be formulated into a liquid sustained release drug delivery system. In particular embodiments, the resulting aqueous gel is viscous with adhesive characteristics suitable for granulation or extrusion/spheronization. In other particular embodiments, the drug is distributed with content uniformity within the aqueous gel. In yet other particular embodiments, the electrostatic interaction between the drug and ion-exchange matrix and/or permeable membrane influences the rate and extent of drug release. It is also contemplated that the aqueous gel can be used in an aqueous coating process to layer drug on a sugar sphere, forming a bead. The gel can also be dried to produce hydrophilic drug complex that can be easily reconstituted with rapid dissolution. The methods contemplated by the instant invention can be easily scaled up for large-scale manufacturing. As such, the instant invention also encompasses novel drug-loaded beads formed from an aqueous gel, having excellent content uniformity and high hydrophilicity, readily produced in large quantities.

In the above embodiments, the invention also encompasses dispersing the ion-exchange matrix drug complex into a dispersion medium that is substantially free of diffusible counterions.

In another embodiment, the dispersion medium further comprises a highly hydrated excipient that attracts water in the dispersion medium.

In another embodiment, the dispersed phase is membrane-coated. In particular embodiments, the membrane is polymeric and can be porous or non-porous. In a particular preferred embodiment, the membrane controls diffusion of the drug.

In another embodiment, the interaction between the ion-exchange matrix and drug controls release of the drug and/or the amount of free drug in the dispersion medium.

In yet another embodiment, the dispersion medium comprises a polyelectrolyte having the same charge as the electrolytic drug and the dispersed phase is membrane-coated, wherein diffusion of the drug and any swelling of the ion-exchange drug complex is controlled by Donnan membrane effects.

The present invention also relates to methods for treating a patient suffering from a symptom or condition. In such embodiments, the invention relates to methods for treating a condition or symptom, comprising administering a liquid form controlled release drug composition as described in the present invention to a patient in need thereof.

In a specific embodiment, the base form of albuterol is allowed to interact with alginic acid in the presence of water, surprisingly forming a physically stable aqueous gel that can be used in forming drug-loaded beads. In a more preferred embodiment, the drug-loaded beads further comprise lactose. In other embodiments, the beads can optionally comprise stearic acid or other excipients that can facilitate extrusion. In certain preferred embodiments, the resulting beads are coated with EUGRAGIT® and suspended in a dispersion medium that comprises water and sucrose. In particularly preferred embodiments, the dispersion medium comprises Syrup NF. The dispersion medium can also further comprise preservatives and other non-active additives. In such embodiments, the resulting liquid sustained release product is capable of maintaining physical stability in a bottle and capable of achieving controlled release of drug product when administered to a patient.

The present invention can be understood more fully by reference to the following detailed FIGS., description and illustrative examples, which exemplify non-limiting embodiments of the invention.

### 4. BRIEF DESCRIPTION OF THE DRAWINGS

FIG. 1 shows one embodiment of the invention, wherein in the liquid form controlled drug release comprises a dispersed phase 1 comprising a calcium alginate matrix drug complex that is coated with a porous diffusion-controlling membrane 5.

FIG. 2 shows a process for release of the electrolytic drug from a dispersed phase 1 comprising a calcium alginate matrix drug complex that is coated with a porous diffusion-controlling membrane 5.

FIG. 4 shows Langmuir interaction isotherms at 25°C resulting from the interaction of propranolol with the ion-exchange matrices sodium alginate (◆), xanthan gum (■) and gellan gum (●).

FIG. 5 shows Scatchard plots at 25°C resulting from the interaction of propranolol with the ion-exchange matrices sodium alginate (◆), xanthan gum (■) and gellan gum (●).

FIG. 6 shows a method for preparing calcium alginate in the form of beads by controlled addition of an aqueous suspension of sodium alginate to an aqueous solution of CaCl₂.

FIG. 7 shows a multilayered calcium alginate drug precursor that forms a calcium alginate drug complex when contacted with water.

FIG. 8 shows a diagram of a miniature fluid bed coater.

FIG. 9 shows the release profile of albuterol from coated beads.

FIG. 10 shows the release profile of albuterol in various coated albuterol/alginate/lactose bead suspensions.

FIG. 11 shows the release profile of albuterol in various coated albuterol/carbopol/lactose bead suspensions.

FIG. 12 shows the effect of dispersion medium components on albuterol release from sustained release suspensions.

FIG. 13 shows the effect of lactose level in bead formulation on albuterol release from sustained release suspensions.

FIG. 14 shows the effect of coating level on albuterol release from sustained release suspensions.

FIG. 15 shows the release profile of albuterol from various coated beads in suspension released in water.

FIG. 16 shows the difference in albuterol release from various coated beads in suspension released in water as compared to release in typical buffer systems.

### 5. DETAILED DESCRIPTION OF THE INVENTION

### 5.1 DEFINITIONS

As used herein, the term "patient" includes, but is not limited to any animal classified as a mammal, including humans, domestic and farm animals, and zoo, sports and pet companion animals such as household pet and other domesticated animals such as, but not limited to, cattle, sheep, ferrets, swine, horses, poultry, rabbits, goats. As used herein, the terms "subject" and "patient" can also be used interchangeably. A patient is preferably a mammal such as a non-primate (*e.g.*, cows, pigs, horses, cats, dogs, rats, etc.) and a primate (*e.g.,* monkey and human).

As used herein, the terms "treat" and "treatment" refer to both therapeutic treatments and prophylactic or preventative measures, wherein the object is to prevent or attenuate an undesired physiological condition, disorder or disease or obtain beneficial or desired clinical results. For purposes of this invention, beneficial or desired clinical results include but are not limited to, alleviation of symptoms; diminishment of extent of condition, disorder or disease; stabilized (*i.e.,* not worsening) state of condition, disorder or disease; delay or slowing of condition, disorder or disease progression; amelioration of the condition, disorder or disease state; remission (whether partial or total), whether detectable or undetectable; or enhancement or improvement of condition, disorder or disease. Treatment includes eliciting a cellular response that is clinically significant, without excessive levels of side effects. Treatment also includes prolonging survival as compared to expected survival if not receiving treatment.

As used herein, the phrase "electrolytic drug" refers to the pharmaceutically acceptable ionic form of a drug that is capable of being ionized by dissociation or protonation.

As used herein, the term "diffusible counterion" refers to a pharmaceutically acceptable ion that is capable of displacing or replacing the electrolytic drug from the ion-exchange matrix. If the diffusible counterion has a positive charge, it is referred to herein as a diffusible counter-cation. Non-limiting examples of diffusible counter-cations such as, *e.g.,* sodium, potassium, magnesium or calcium. If the diffusible counterion has a negative charge, it is referred to herein as a diffusible counter-anion. Non-limiting examples of diffusible counter-anions include, *e.g.,* chloride, bromide, iodide, and phosphate.

As used herein, the term "water soluble" when used in connection with an electrolytic drug means having a solubility of greater than about 3 g of the electrolytic drug in 100 ml of water at any physiologically relevant pH. In particular embodiments, the term water soluble means having a solubility of greater than 1 g of the electrolytic drug in 100 ml of water at any physiologically relevant pH.

As used herein, the phrase "substantially free of diffusible counterions" when used to describe the concentration or presence of diffusible counterions in the dispersion medium means the concentration of diffusible counterions in the dispersion medium is less than about 0.1-0.5 moles per liter of liquid, preferably less than about 0.05-0.1 moles per liter of liquid, more preferably less than about 0.01-0.5 moles per liter of liquid, most preferably less than about 0.01 moles per liter of liquid.

As used herein, the phrase "base form of the amine" when used in connection with a drug or an ion-exchange matrix means that substantially all the amine-nitrogen atoms are unprotonated and have a neutral charge.

As used herein, the phrase "acid form" when used in connection with a drug or an ion-exchange matrix means that substantially all the acid groups are in their undissociated, uncharged acid form.

As used herein, the term "polyelectrolyte" means a molecule having a charge on its surface. In certain embodiments, a polyelectrolyte has a molecular weight large enough that it will not substantially diffuse through the optional diffusion-controlling membrane such as those useful for the present invention. The term "polyelectrolyte" encompasses molecules, oligomers, co-oligomers, polymers, co-polymers, each of which may be organic, inorganic or a combination thereof.

As used herein, the term "hydrophilic colloid" encompasses large organic molecules capable of being solvated or associated with the molecules of the dispersion medium. Hydrophilic colloid also encompasses a colloidal dispersion in which the dispersed particles are more or less liquid and exert a certain attraction on and adsorb a certain quantity of the fluid in which they are suspended.

As used herein, the term "diffuse double layer" means the molecularly dynamic region in the immediate vicinity of the charged surface of a dispersed or suspended solid or liquid phase, such dispersed material usually having a particle size that falls within the colloidal range, that contains both ions with opposite and similar charge to that of the colloidal surface, but possessing overall, in the extent of the diffuse double layer, an excess concentration of oppositely charged ions (*i.e.,* counterions) sufficient to neutralize the surface charge of the dispersed phase.

As used herein, the phrase "uncomplicated gel" refers, in part, to the absence of spectator ions in an aqueous gel.

As used herein, the phrase "highly hydrated" describes a component having sufficient hydrogen bonds to restrict the thermodynamic activity of water.

### 5.2 THE DOSAGE FORM OF THE INVENTION

As provided above, the invention described and claimed herein comprises a novel class of sustained release drug formulations comprising a drug confined in a dispersed phase of a suspension by an ion-exchange matrix having a surface charge opposite that of the drug.

The invention specifically encompasses liquid sustained release formulations comprising drug-loaded beads produced by combining an ion-exchange matrix that is a hydrophilic colloid and a drug having a charge opposite that of the matrix in the presence of water.

The liquid sustained release formulations of the invention are capable of controlled release spanning about 8 hours, about 12 hours, about 18 hours, most preferably about 24 hours, up to about 48 hours.

In certain embodiments, the liquid sustained release drug delivery systems comprise beads which contain a drug and are coated with a material that controls the release of the drug. Preferably the coating is a barrier through which the drug must diffuse before it becomes bioavailable.

In certain embodiments, the drug is in its base form. The hydrophilic colloids contemplated by the instant invention have acidic and basic functional groups which can be ionized under certain conditions which leads to extensive hydration characterized by strong ion-dipole interactions with water. As such the ion-exchange matrix interaction with the drug results in spontaneous and reversible formation of uncomplicated aqueous gels. Such an interaction enhances thermodynamic stability by virtue of attractive electrostatic forces. Other advantages conferred by such interactions include but are not limited to high viscosity and electrophoretic mobility. The aqueous gel resulting from the interaction is suitable for granulation and spheronization/extrusion. In preferred embodiments, the resulting gel is used to form beads. The resulting drug-loaded beads are very easily manufactured with few critical variables that require exact control. The beads are hydrophilic with content uniformity. The beads are uniquely suitable for incorporation into sustained release liquid suspension dosage forms. A suitable polymer coating on the bead can confine the drug inside the bead because of electrical neutralizing constraints associated with the inability of the drug complex to permeate the membrane. The drug remains associated with the ion-exchange matrix until encountering other components that encourage drug diffusion. As such, in certain embodiments, the dispersed phase is membrane-coated.

In certain embodiments, the dispersion medium is substantially free of diffusible counterions, and optionally includes a polyelectrolyte with the same charge as the drug in a dispersion medium. Drug "release" is triggered when the suspension is placed in an environment, for example gastric or intestinal fluid, with a high concentration of water and small ions (*i.e.,* capable of diffusing through and/or displacing the drug from the ion exchange matrix) that possess the same charge as the drug. These small ions or counter ions swamp the ion-exchange matrix drug complex and cause release of the electrolytic drug into the dispersed phase of gastrointestinal fluid. Thus, the concentration of electrolytic drug in the dispersion medium is effected, in part, by the rate and extent to which the ion-exchange matrix influences diffusion into the dispersion medium. The rate and extent to which the ion-exchange matrix influences diffusion can affect the sustained release profile of the drug.

In alternate embodiments, the dispersion medium comprises a component(s) that is highly hydrated and capable of associating with water. Such components attract water from the dispersion medium such that water activity outside the bead and in the dispersion medium is less than inside the bead. In various embodiments, the dispersion medium has low enough water activity to preclude water diffusion into the drug-loaded bead and the development of internal osmotic pressure until the formulation is administered and the dispersion medium is diluted. In preferred embodiments, the component is a non-electrolytic excipient such as, but not limited to, sucrose, dextrose, maltose, manitol, sorbitol, glycerin, or low molecular weight polyethylene glycol. In certain such embodiments, the drug delivery systems contemplated by the invention are activated by water (*e.g.,* water in gastric fluid). In such embodiments, when the activity of water outside the bead in the dispersion medium is greater than the activity of water inside the bead, water will diffuse through the coating and dissolve soluble components of the bead and create a reservoir of diffusible free drug. Such free drug can permeate the coating which can control release of the drug to the patient.

As such, it is an object of the invention to provide such a novel class of drug delivery system comprising a drug confined in a dispersed phase of a suspension, which comprises an ion-exchange matrix with a charge opposite that of the drug.

In certain embodiments, the composition of the present invention, particularly the dispersion medium, also comprises a polyelectrolyte having a surface charge like that of the electrolytic drug. Without being limited by theory, Applicants believe that release of the electrolytic drug from the dispersed phase is controlled or delayed by repulsive interactions between the polyelectrolyte and the electrolytic drug, since both have the same charge. This "trapping" of the electrolytic drug allows for the administration of the ionized form of drugs with high water solubility, and provides a low concentration of the electrolytic drug in the dispersion medium.

In one embodiment, the present invention relates to a liquid form controlled release drug composition, comprising:
(a) a dispersed phase comprising a water-soluble electrolytic drug associated with a pharmaceutically acceptable ion-exchange matrix, wherein the surface charge of the ion-exchange matrix is opposite that of the electrolytic drug; and
(b) a dispersion medium that is substantially free of diffusible counterions.

In one embodiment, the present invention relates to a liquid form controlled release drug composition, comprising:
(a) a dispersed phase comprising a water-soluble electrolytic drug associated with a pharmaceutically acceptable ion-exchange matrix, wherein the surface charge of the ion-exchange matrix is opposite that of the electrolytic drug; and
(b) a dispersion medium comprising a polyelectrolyte having the same charge as the electrolytic drug.

In one embodiment, the present invention relates to a liquid form controlled release drug composition, comprising:
(a) a dispersed phase comprising an ion-exchange matrix drug complex comprising a pharmaceutically acceptable ion-exchange matrix and a water-soluble electrolytic drug associated with the ion-exchange matrix, wherein the surface charge of the ion-exchange matrix is opposite that of the electrolytic drug; and
(b) a dispersion medium that is substantially free of diffusible counterions.

In one embodiment, the present invention relates to a liquid form controlled release drug composition, comprising:
(a) a dispersed phase comprising an ion-exchange matrix drug complex comprising a pharmaceutically acceptable ion-exchange matrix and a water-soluble electrolytic drug associated with the ion-exchange matrix, wherein the surface charge of the ion-exchange matrix is opposite that of the electrolytic drug; and
(b) a dispersion medium comprising a polyelectrolyte having the same charge as the electrolytic drug.

The ion-exchange matrix can be a high molecular weight organic compound such as an oligomer, co-oligomer, polymer or co-polymer; a porous inorganic network solid such as, *e.g.,* a zeolite; and/or combinations thereof, which have charged surfaces and are capable of retaining an oppositely-charged ion. As used herein, the phrase "cation-exchange matrix" refers to an ion exchange matrix which is capable of retaining a cationic form of a drug. As used herein, the phrase "anion-exchange matrix" refers to an ion exchange matrix which is capable of retaining an anionic form of a drug.

The design of the system and the selection of appropriate components are predicated on the charge of the therapeutically active ingredient (drug). The invention is suitable for the administration of drugs which are uncharged bases or acids; or cationic or anionic drugs, which are strong electrolytes as well as weakly acidic drugs above their pKa (anions) and weakly basic drugs below their pKa (cations). When the drug is an ion, the ion-exchange matrix must have a charge opposite that of the drug ion. When the drug is an uncharged base or acid, the ion-exchange matrix is in the form of an acid or base, respectively. If the electrolytic drug is a cation, then an ion-exchange matrix with a negative surface functionality must be utilized as ion-exchange matrix. If the drug is an anion, then an ion-exchange matrix with a positive surface functionality must be employed. Examples of suitable pharmaceutical ingredients that may be used to form the core in this case include but are not limited to chitosan, polylysine, and gelatin.

Cation- and anion-exchange matrices are well-known in the art. Non-limiting examples of useful cation-exchange matrices include cation-exchange resins such as, *e.g.*, resins having polymer backbones comprising styrene-divinyl benzene copolymers and having pendant sulfonate groups, available from Rohm and Haas, Philadelphia, PA, and sold under the tradename AMBERLITE™ IRP69; methacrylic acid and divinyl benzene co-polymers which have a carboxylate functionality, available from Rohm and Haas, and sold under the tradenames AMBERLITE™ IRP64 and IRP88; hydrophilic colloids such as, *e.g.*, alginate, carboxymethylcellulose, croscarmellose, microcrystalline cellulose, xanthan gum, carboxy vinyl polymers such as carbomer 94, gelatin; or any combination thereof. In one embodiment, the cation-exchange matrix is alginate, carboxymethylcellulose, microcrystalline cellulose, xanthan gum, carboxy vinyl polymer, gelatin or any combination thereof.

Non-limiting examples of useful anion-exchange matrices include anion-exchange resins such as, *e.g.,* resins having polymer backbones comprising styrene-divinyl benzene copolymers and having pendant ammonium or tetraalkyl ammonium functional groups, available from Rohm and Haas, Philadelphia, PA, and sold under the tradename DUOLITE™ AP143; and hydrophilic colloids such as, but not limited to, chitosan, polylysine, or gelatin; and any combination thereof. In one embodiment, the anion-exchange matrix is chitosan, polylysine, gelatin or any combination thereof.

In certain embodiments, the ion-exchange matrix is water-insoluble. In an alternate and preferred embodiment, the ion-exchange matrix is water soluble. In such embodiments, the ion-exchange matrix is capable of being solvated with the dispersion medium, and is preferably a hydrophilic colloid. The invention contemplates hydrophillic colloids including but not limited to natural materials such as starch, agar, cellulose, alginic acid, guar gum, xanthan gum, gelatin, acacia, and albumin have been used for applications that range from something as simple as a wet binder to something as novel as a component of microspheres. Non-limiting synthetic examples include methylcellulose, carboxymethylcellulose, hydroxypropylmethyl cellulose, methylacrylic acid, polylactic acid, polyglycolic acid, and polyanhydrides are also widely deployed in non-limiting applications ranging from the traditional, such as wet granulation, to the more contemporary, such as functional coatings and biodegradable implants.

In preferred embodiments, the cation-exchange matrix is a hydrophillic colloid. In such embodiments, the cation-exchange matrix is alginate, carboxymethylcellulose, microcrystalline cellulose, xanthan gum, carboxyvinyl polymers such as carbomer 94, or any combination thereof. In certain embodiments, the hydophilic colloid is cross-linked to reduce swelling. In a particularly preferred embodiment, the ion-exchange material is calcium alginate.

In other preferred embodiments, the anion-exchange matrix is a hydrophillic colloid. In such embodiments, the anion-exchange matrix is chitosan, polylysine, gelatin, or any combination thereof. In certain embodiments, the hydophilic colloid is cross-linked to reduce swelling.

The water soluble electrolytic drug associates with the ion-exchange matrix and forms an ion-exchange matrix drug complex. Without being bound by any particular theory, Applicants believe that one advantage of the present invention stems from the electrostatic interactions between the drug and the ion-exchange matrix, which circumvents many of the traditional challenges faced when formulating liquid sustained release oral dosages.

In certain embodiments, the ion-exchange matrix drug complex is in the form of a particulate or bead. The particulate or bead is of a size which can be administered orally in a liquid dosage form. In one embodiment, the particulate or bead is of a size and/or density such that it does not settle in suspension. Preferably, the particulate or bead does not have undesirable patient attributes. In particular embodiments, the diameter of the particulate or bead ranges from about 0.01 µm to about 2000 µm; in another embodiment, from about 0.1 µm to about 1000 µm; and in another embodiment, from about 1 µm to about 1000 µm. In other embodiments, the diameter of the particulate or bead is greater than 2000 µm, greater than 3000 µm, or greater than 5000 µm. In alternate embodiments, the diameter of the particulate or bead is no greater than 2000 µm, no greater than 1000 µm, preferably no greater than 500 µm, more preferably no greater than 50 µm, most preferably no greater than 1 µm.

The core may further comprise pharmaceutically acceptable processing aid useful for forming solid dosage forms including, but limited to, bulking agents such as starch, titanium oxide, and silica; preservatives; stabilizers such as antioxidants; lubricants such as vegetable oils; and the like.

In a preferred embodiment, the ion-exchange matrix drug complex further comprises a low molecular weight, soluble, non-electrolytic excipient. Such an excipient is capable of dissolving in water and diffusing out of the bead when the bead absorbs water and thereby reduces osmotic pressure inside the bead. The excipient must have a low enough molecular weight to permeate any membrane coating the bead. In various embodiments, the amount of excipient included in the bead can affect the rate of drug release. In various embodiments, the excipient is present in the bead at about 5% to about 10%, at about 10% to about 20%, at about 20% to about 30% at about 30% to about 40%, at about 40% to about 45%. In a specific preferred embodiment, the excipient is lactose. In certain such embodiments, the more lactose incorporated in the dispersed phase during manufacturing, the faster the release of drug from the bead after administration. In various embodiments, lactose is present in the bead at about 5% to about 10%, at about 10% to about 20%, at about 20% to about 30% at about 30% to about 40%, at about 40% to about 45%. In certain preferred embodiments, lactose is present in the bead at about 20% to about 30%. Other examples of soluble non-electrolytic excipients encompassed by the invention include but are not limited to dextrose, maltose, manitol, sorbitol, glycerin, or low molecular weight polyethylene glycol.

In one embodiment, the ion-exchange matrix drug complex further comprises a diffusion-controlling membrane coating. The membrane coating is useful for further controlling diffusion of counterions into and drug out of the ion-exchange matrix. Thus, the diffusion-controlling membrane coating is useful for controlling the release of the electrolytic drug into the dispersion medium and/or the digestive tract after administration to a patient. The invention encompasses the use of any membrane-coating that provides diffusion control. The coating materials may be any of a large number of natural or synthetic film-formers used alone, in admixture with each other, and in admixture with other components such as plasticizers, pigments, and other substances. The components of the coating are preferably insoluble in, and permeable to, water. Incorporation of a water-soluble substance can be useful in altering the permeability of the coating. Diffusion-controlling membranes are known in the art. Non-limiting examples include ethylcelluloses such as SURELEASE® (Colorcon, Westpoint, PA); methylmethacrylate polymers such as EUDRAGTT® (Röhm Pharma, GmbH, Weiterstat, Germany); cellulose esters, cellulose diesters, cellulose triesters, cellulose ethers, cellulose ester-ether, cellulose acylate, cellulose diacylate, cellulose triacylate, cellulose acetate, cellulose diacetate, cellulose triacetate, cellulose acetate propionate, and cellulose acetate butyrate. In a preferred embodiment, the coating is a methylmethacrylate polymer.

In one embodiment, the diffusion-controlling membrane is selected from the group consisting of ethylcellulose, methylmethacrylate, cellulose esters, cellulose diesters, cellulose triesters, cellulose ethers, cellulose ester-ether, cellulose acylate, cellulose diacylate, cellulose triacylate, cellulose acetate, cellulose diacetate, cellulose triacetate, cellulose acetate propionate, cellulose acetate butyrate, and combinations thereof.

In one embodiment, the ion-exchange matrix drug complex is coated with from about 1% up to about 75% of diffusion-controlling membrane based on the total weight of the ion-exchange matrix drug complex and the diffusion-controlling membrane; in another embodiment, from 5% to about 50%; and in a preferred embodiment, from about 10% to about 30%. Typically, the more coating, the more delay in the release of the drug.

In one embodiment, drug-loaded alginate beads are coated with sufficient EUDRAGIT® (Rohm) RS 30 D to provide a coated bead having from about 20% to about 30% by weight of coating based on the total weight of the coating and the drug-loaded alginate beads.

Preferably, the dispersion medium of the invention is a liquid with a low concentration of diffusible counterions. In a preferred embodiment, the dispersion medium is substantially free of diffusible counterions. Useful liquids include non-aqueous pharmaceutically acceptable liquids, water, or a combination thereof. The only requirement of the dispersion medium is that it has a low concentration of diffusible counterions. As used herein, the term "low" when used in connection with the diffusible counterion concentration in the dispersion medium means less than about 0.1-0.5 moles per liter of liquid, preferably less than about 0.05-0.1 moles per liter of liquid, more preferably less than about 0.01-0.5 moles per liter of liquid, most preferably less than about 0.01 moles per liter of liquid. In certain embodiments, the diffusible counterion concentration is less than 0.001-0.1 moles per liter of liquid, preferably no greater than 0.01 moles per liter of liquid. In certain embodiments, the dispersion medium is substantially free of diffusible counterions.

Because the concentration of diffusible counterions in the dispersion medium is low, the extent of counterion exchange with the electrolytic drug is minimized or eliminated. Thus, a substantial fraction of the electrolytic drug remains associated with the ion-exchange matrix, *i.e.,* in the dispersed phase. In one embodiment, the amount of free drug in the dispersion medium is less than 10% based on the total molar amount of drug in the dispersion medium and dispersed phase; in another embodiment, the amount of free drug in the dispersion medium is less than 5% based on the total molar amount of drug in the dispersion medium and dispersed phase; and in another embodiment, the amount of free drug in the dispersion medium is less than 0.5% based on the total molar amount of drug in the dispersion medium and dispersed phase; and in yet another embodiment, the amount of free drug in the dispersion medium is less than 0.05% based on the total molar amount of drug in the dispersion medium and dispersed phase.

As noted above, in specific embodiments, the liquid form controlled release drug composition comprises a dispersion medium further comprising a polyelectrolyte having the same charge as the electrolytic drug. Preferably, the polyelectrolyte does not displace the drug from the ion-exchange matrix. Rather, the polyelectrolyte further confines the drug in the matrix until the formulation is exposed to a high concentration of water and small ions that possess the same charge as the drug. Preferably, the polyelectrolyte is also a pharmaceutically acceptable molecule incapable of diffusing through a membrane. Preferably the polyelectrolyte is capable of counteracting the effects of any diffusible counterions that may be present in the dispersion medium.

In certain preferred embodiments, the dispersion medium further comprises a high concentration of excipient(s) that are highly hydrated, capable of associating with the water in the dispersion medium. Although the drug is highly soluble in aqueous dispersion media, the presence of the highly hydrated component in the dispersion medium attracts the water in the dispersion medium necessary to begin the dissolution of drug from the drug-ion-exchange matrix complex. Only until the formulation is administered and gastric liquids (largely water) dilute the dispersion medium will drug dissolve and become available and begin to permeate the membrane and/or diffuse from the bead. In such embodiments, the dispersion medium is substantially devoid of free drug, preferably less than 0.5% drug, most preferably less than 0.05% drug, is in the dispersion medium. In various embodiments, the highly hydrated component is present in the dispersion medium, on a weight to weight basis, at about 10% to about 20%, at about 20% to about 30%, at about 30% to about 40%, at about 40% to about 50%, at about 50% to about 60%. Preferably, the component is present at about 60% to about 65%, up to about 70%. In specific preferred embodiments, the dispersion medium comprises sucrose, or other sugar molecules. In such embodiments, the dispersion medium is, on weight to weight basis, more than 10% sucrose, preferably more than 20% sucrose, more preferably more than 30% sucrose, even more preferably more than 40% sucrose, and most preferably more than 50%. In a most preferred embodiment, the dispersion medium comprises about 65% sucrose (*i.e.,* Syrup NF), no more than about 70%. Other examples of excipients encompassed by the invention include but are not limited to dextrose, manitol, fructose, polyethylene glycol, glycols, and glycerins. One of skill in the art could readily determine other highly hydrated excipients that would function similarly.

The inventors have recognized that in preparing liquid dosage forms, components necessary for dosage characteristics such as but not limited to palatability and suspension can be chosen in order to keep the concentration of diffusible counterions low and to choose components that are polyelectrolytes capable of further trapping the drug in the ion-exchange matrix drug complex.

Non-limiting examples of useful positively-charged polyelectrolytes include oligomers and polymers comprising the ammonium and/or tetralkylammonium salt forms of alkylaminoethyl(meth)acrylate, dimethylaminoethyl(eth)acrylate, aminoethyl(meth)acrylate, dimethylaminoethyl(meth)acrylate, dimethylaminopropyl(meth)acrylamide, vinyl pyridine, vinyl-N-ethylpyridine, vinylbenzyldimethylammine; or a positively charged hydrophillic colloid such as. *e.g.,* chitosan; and any combination thereof. In a specific embodiment, the positively-charged polyelectrolyte is chitosan.

Non-limiting examples of useful negatively-charged polyelectrolytes include oligomers and polymers comprising the salt form of (meth)acrylic acid, (eth)acrylic acid, itaconic acid, maleic acid anhydride, vinylsulfonic acid, vinyl sulfuric acid, styrenesulfonic, vinylphenylsulfuric acid, alginate, xanthan gum, carboxymethyl cellulose, carboxymethyl-hydroxyethyl cellulose, dextran sulfate, hyaluronic acid, heparin, chondroitin sulfate, galacturonic acid, glutamic acid, gellan gum and any mixture thereof. In a specific embodiment, the negatively-charged polyelectrolyte is xanthan gum.

In a preferred embodiment, the polyelectrolyte molecules do not further comprise diffusible counterions.

The inclusion of a like-charged, high molecular weight, polyelectrolyte in the dispersion medium, which is not capable of diffusing through the membrane, forces the lower molecular weight drug into the dispersed phase (through Donnan membrane effect) where it is confined by electrostatic force.

The liquid form controlled release drug composition can further comprise a dispersion additive selected from the group consisting of stabilizing agents, dispersing agents, and the like, provided the excipients do not adversely affect the intended operation of the invention.

The liquid form controlled release drug composition can further comprise excipients useful in oral liquid dose formulations such as, *e.g.,* sweetening agents, flavoring agents, coloring agents, thickeners, and the like, provided the excipients do not adversely affect the intended operation of the invention.

The present invention contemplates the use of the dispersion additives and excipients discussed in the above embodiments that are also polyelectrolytes having a similar charge as the electrolytic drug. The invention also encompasses the use of such dispersion additives and excipients discussed in the above embodiments as the non-drug components included in the bead to reduce osmotic pressure or in the dispersion medium to impede drug dissolution prior to administration.

Advantages of the dosage form of the present invention is that the ion-trapping, osmotic control, and thermodynamic balancing mechanisms are generally applicable and inherently stable and the fact that effects can be implemented through the use of traditional and widely accepted pharmaceutical excipients one skilled in the art could utilize.

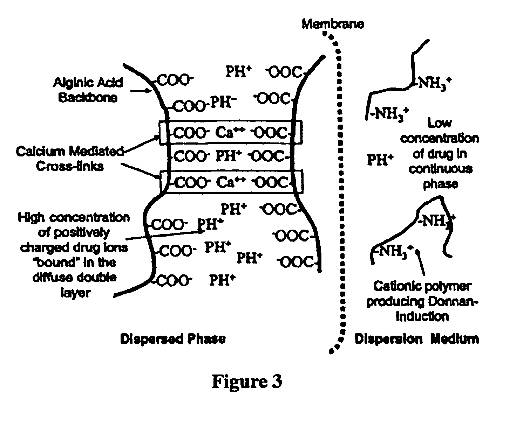

For illustration, the example of a cation-forming drug (propranolol) employing an alginate core bead will be used as a specific example for each stage, but the overall objective is to create polyelectrolyte(s) with a surface charge selected so as to confine oppositely charged drug molecules in the diffuse double layer ("DDL") of the colloidal dispersed phase as depicted in FIG. 3. Typically, the dispersed phase will be aggregated in the form of an approximately spherical particle with a diameter in the 100 to 2000 um range. The size of the particles and/or swelling can be restricted through the use of cross-linking and/or cross-linking agents. In various embodiments, cross-linking can be accomplished through covalent bonding, *e.g.,* AcDiSol is cross-linked carboxymethyl cellulose and cross-linked gelatin can be achieved via the exposure of gelatin to formaldehyde.

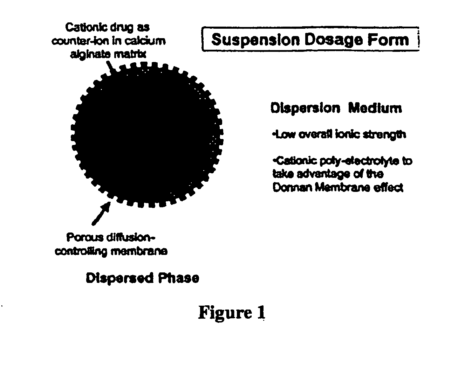

One embodiment of the invention is depicted in FIG. 1. An electrolytic or ionic form of a drug is confined in a dispersed phase of a suspension 1 comprising an ion exchange matrix with a charge opposite that of the drug 2. The dispersion medium 3 comprises a polyelectrolyte 4 with a surface charge the same as that of the drug. A thermodynamically stable trapping of water-soluble electrolytic drug in the dispersed phase 1 of the suspension 2 is achieved using electrostatic interactions, allowing a water-soluble drug to be confined in the dispersed phase 1 at high concentration without diffusing into the dispersion medium 3. A reservoir is created in which the drug will remain in the dispersed phase 1 until release is triggered by a high concentration of ions similar in charge to the drug in the dispersed phase. This triggering occurs when the drug is administered into physiological fluids with relatively high ionic strength. When the dispersed phase 1 comprises an optional diffusion-controlling membrane coating 5, the rate of release can be further controlled, because the rate of counterion exchange and electrolyte diffusion is inhibited.

Also by way of illustration, the exemplary drug albuterol will be used to demonstrate the applicability of a drug delivery system comprising drug-loaded beads manufactured from a physically stable aqueous gel that incorporates lactose. Typically such beads are then suspended in a dispersion medium containing sucrose. However, the overall objective is to create a stable aqueous gel by reacting any charged drug with an ion-exchange matrix having a charge opposite that of the drug in the presence of water to form beads that further comprise a soluble non-electrolyte capable of dissolving and diffusing out of the bead to relieve the osmotic pressure in the beads. Typically such beads are coated with a polymeric membrane that contributes to the control of drug release and the beads are suspended in a dispersion medium that includes a non-electrolytic excipient that is highly hydrated and capable of ensuring that the water activity outside the beads in the dispersion medium is less than the water activity inside the bead.

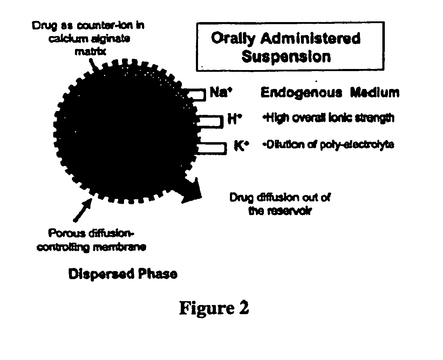

In the present system, the selection of a polyelectrolyte with a surface charge the same as the drug can serve to further depress free drug concentration in the dispersion medium. This occurs because of the Donnan membrane effect, where the transfer of the drug ion is inhibited by repulsion with the liked charged polyelectrolyte *(see* M. R. Franklin et al. Drug Absorption, Action, and Disposition in 57 Remington: The Science and Practice of Pharmacy 1118 (A.R. Gennaro, ed. 2000)) The fact that the polyelectrolyte has high molecular weight means that it can not diffuse through the diffusion controlling membrane on the drug-loaded bead. As a result, to maintain constant activity across the membrane, the diffusible drug is pushed into the bead. Thus, the membrane controls both the influx of swamping electrolyte and the efflux of drug. This mechanism is illustrated in FIG. 2. for a dispersed phase 1 comprising a calcium alginate matrix and a cationic electrolytic drug. After oral administration, the dispersion medium 3 is admixed with biological fluids having a high concentration of counter-cations such as sodium, potassium and proton. The counter-cations penetrate the diffusion-controlling membrane coating 5 and displace the electrolytic drug associated with the calcium alginate. The electrolytic drug then moves from an area of high concentration (the dispersed phase 1), crosses the diffusion-controlling membrane coating 5, and moves to an area of low drug concentration (the dispersion medium 2). Thus, the liquid dosage, thermodynamically stable, drug suspension possessing low free drug concentration in the dispersion medium, provides sustained drug release when administered to a patient. If chosen judiciously, the inclusion of a polyelectrolyte in the dispersion medium of a suspension can also serve the traditional purpose of increasing viscosity and slowing sedimentation.

The amount of polyelectrolyte used in the liquid form controlled release drug composition depends on a several factors such as but not limited to, *e.g.,* the amount and type of electrolytic drug in the ion-exchange matrix drug complex; the type of ion-exchange matrix; the type of porous diffusion-control membrane, if any, used; the type of liquid used in the dispersion medium; and the charge density on the polyelectrolyte. In one embodiment, the polyelectrolyte content ranges from about 0.1 molar equivalents of ionic charge to about 10 molar equivalents of ionic charge per molar equivalent electrolyte drug. As used herein, the phrase "molar equivalents of ionic charge" refers to the number of salt sites having the same charge as the electrolytic drug. In another embodiment, the polyelectrolyte content ranges from about 0.5 molar equivalents of ionic charge to about 5 molar equivalents of ionic charge per molar equivalent electrolyte drug. In another embodiment, the polyelectrolyte content ranges from about 1 molar equivalents of ionic charge to about 3 molar equivalents of ionic charge per molar equivalent electrolyte drug.

The drugs useful in the embodiments of the present invention, and their recommended usage are known in the art and have been described in such literature as the Physician's Desk Reference (56th ed., 2002). One skilled in the art could readily determine the electrolytic drugs that would be particularly suitable for the dosage forms contemplated by the present invention.

The electrolytic drugs useful in the invention include but are not limited to, *e.g*., cardiovascular drugs, beta-agonists, respiratory drugs, sympathomimetic drugs, cholinomemetic drugs, adrenergic drugs, antimuscarinic and antispasmodic drugs, skeletal muscle relaxants, diuretic drugs, anti-migraine drugs, anesthetics, sedatives and hypnotics, antiepileptics, psychopharmacologic agents, analgesics, including opioid and non-opioid analgesics, antipyretics, CNS stimulants, antineoplastic and immunosuppressive drugs, antimicrobial drugs, antihistamines, anti-inflammatories, antibiotics, decongestants, cough suppressants, expectorants, and the like.

Non-limiting examples of cationic-active agents useful in the present include the pharmaceutically acceptable salts of acetophenazine, albuterol, amitriptyline, benztropine, biperiden, bitolerol, bromodiphenhydramine, brompheniramine, buprenorphine, carbinoxamine, chlorcyclizine, chlorpheniramine, chlorphenoxamine, chlorpromazine, clemastine, clonidine, codeine, cyclobenzaprine, cyclizine, cyclobenzaprine, cyproheptadine, desipramine, dexbrompheniramine, dexchlorpheniramine, dextroamphetamine dibucaine, dextromethorphan, dicyclomine, diethylpropion, dihydroengotamine, diltiazem, diphemanil, diphenhydramine, doxepin, doxylamine, ergotamine, fluphenazine, haloperidol, hydrocodone, hydroxychloroquine, hydroxyzine, hyoscyamine, imipramine, isoproterenol, levopropoxyphene, lidocain, maprotiline, meclizine, mepenzolate, meperidine, mephentermine, mesoridazine, metaproterenol, methadone, methdilazine, methscopolamine, methysergide, metoprolol, morphine, nalorphine, nortriptyline, noscapine, nortriptylenepyrilamine, nylindrin, orphenadrine, papaverine, pentazocine, phendimetrazine, phentermine, phenylpropanolamine, phenmetrazine, phenelzine, pibuterol, procaine, prochlorperazine, promazine, propoxyphene, propanolol, protriptyline, pseudoephedrine, pyridostigmine, pyrilamine, quinidine, salmeterol, scopolamine, terbutaline, tetracaine, tranylcypromine, trihexyphenidyl, trimeprazine, tripelennamine, triprolidine and verapamil. In a preferred embodiment, the cationic active agent is an analgesic selected from the group consisting of codeine and morphine. In a preferred embodiment, the cationic active agent is an analgesic such as codeine or morphine.

Non-limiting examples of anionic active agents useful in the present invention include the pharmaceutically acceptable salts of acetylsalicylic acid, cromolyn,diclofenac, diclofenac, diflunisal, ethacrynic acid, fenoprofen, fentanyl, flurbiprofen, furosemide, gemfibrozil, ibuprofen, indomethacin, indoprofen, ketoprofen, naproxen, pentobarbital, phenytoin, salicylamide, salicylic acid, secobarbital, sulindac, thiopental, theophylline and valproate.

In certain embodiments, useful drugs also include the neutral forms of the above mentioned electrolytic drugs which form ions upon association or reaction with the ion-exchange matrix.

In various embodiments, the ion-exchange matrix drug complex comprises ion-exchange matrix in an amount sufficient to convert the drug into ionic form. Optionally, the ion-exchange matrix drug complex comprises ion-exchange matrix in an amount more than sufficient to convert the drug into ionic form.

Also contemplated is the use of two or more electrolytic drugs in the dispersed phase of the liquid form controlled release compositions of the invention.

Still further contemplated is the use of one or more non-electrolytic drugs in the dispersion medium.

### 5.3 METHODS FOR MAKING THE LIQUID DOSAGE FORMS

The present invention also relates to methods for making the liquid sustained release drug formulations contemplated by the present invention.

In one embodiment, the present invention relates to a method for making a liquid sustained release drug formulation, comprising:
(a) providing an ion-exchange resin matrix;
(b) allowing the drug to associate with the ion-exchange matrix to form an ion-exchange matrix drug complex;
(c) drying the ion-exchange matrix drug complex; and
(d) combining the ion-exchange matrix drug complex with a dispersion medium.

In one embodiment, the dispersion medium of step (d) is substantially free of diffusible counterions.

In another embodiment, the dispersion medium of step (d) further comprises a polyelectrolyte.

In another embodiment, step (d) further comprises adding a polyelectrolyte having the same charge as the electrolyte drug to the dispersion medium.

In another embodiment, step (c) further comprises coating the ion-exchange matrix drug complex with a porous diffusion-controlling membrane.

Each of the above steps (a)-(d) influences the nature and performance of the finished product. In some situations, the drug loading step may be conducted after the core beads have been created. '

In one embodiment, the method involves the formation of an aqueous gel.

In another embodiment, the method involves incorporation of a low molecular weight, non-electrolytic, soluble excipient in the formation of the dispersed phase in steps (a) and (b).

In another embodiment, the dispersion medium of step (d) further comprises adding a highly hydrated excipient capable of ensuring greater water activity outside the dispersed phase in the dispersion medium than inside the dispersed phase.

In one embodiment, the present invention relates to a method for making a liquid sustained release drug formulation, comprising:
(a) providing an ion-exchange resin matrix;
(b) allowing the drug to associate with the ion-exchange matrix in the presence of water to form an ion-exchange matrix drug complex, wherein the ion-exchange matrix drug complex is an aqueous gel;
(c) drying the ion-exchange matrix drug complex; and
(d) combining the ion-exchange matrix drug complex with a dispersion medium.

In one embodiment, the dispersion medium of step (d) is substantially free of diffusible counterions.

In another embodiment, the dispersion medium of step (d) further comprises a polyelectrolyte.

In another embodiment, step (d) further comprises adding a polyelectrolyte having the same charge as the electrolyte drug to the dispersion medium.

In another embodiment, step (c) further comprises coating the ion-exchange matrix drug complex with a porous diffusion-controlling membrane.

Each of the above steps (a)-(d) influences the nature and performance of the finished product. In some situations, the drug loading step may be conducted after the core beads have been created.

In another embodiment, the method involves incorporation of a low molecular weight, non-electrolytic, soluble excipient in the formation of the dispersed phase in steps (a) and (b).

In another embodiment, the dispersion medium of step (d) further comprises adding a highly hydrated excipient capable of ensuring greater water activity outside the dispersed phase in the dispersion medium than inside the dispersed phase.

In one embodiment, the invention relates to a method for preparing a liquid form controlled release drug composition, comprising:
(a) allowing a water-soluble electrolytic drug to associate with an ion-exchange matrix to form an ion-exchange matrix drug complex; and
(b) dispersing the ion-exchange matrix drug complex into a dispersion media comprising a polyelectrolyte; wherein
   the surface of the ion-exchange matrix has a charge opposite that of the electrolytic drug; and
   the polyelectrolyte has the same charge as that of the electrolytic drug.

In another embodiment, the present invention relates to a method for preparing a liquid form controlled release drug composition, comprising:
(a) allowing the acid form of an acid-functional ion-exchange matrix to associate with the base form of an amine-based drug to form an ion-exchange matrix drug complex; and
(b) dispersing the ion-exchange matrix drug complex into a dispersion media comprising a polyelectrolyte; wherein
   the polyelectrolyte has a positive charge.

In another embodiment, the present invention relates to a method for preparing a liquid form controlled release drug composition, comprising:
(a) allowing the acid form of an acid-functional ion-exchange matrix to associate with the base form of an amine-based drug in the presence of water to form an ion-exchange matrix drug complex, wherein the ion-exchange matrix drug complex is an aqueous gel;
(b) forming beads using the aqueous gel;
(c) coating the beads with a membrane; and
(d) dispersing the beads into a dispersion media.

In certain embodiments, step (a) of the above method further includes the addition of a non-electrolytic soluble component having a molecular weight low enough to diffuse through the membrane. Preferably, the component is lactose.

In certain embodiments, the dispersion media of step (d) further includes the addition of a highly hydrated excipient capable of associating with water such that the water activity outside the beads in the dispersion media will be less than the water activity inside the beads.

An advantage of preparing the ion-exchange matrix drug complexes as aqueous gels is that spectator ions are absent from the complexes. A formulation based on an aqueous gel can comprise as few as one additional excipient and water. The resulting product formed from the aqueous gel is very hydrophilic with excellent content uniformity. Moreover, electrical neutrality constraints allow drug to be trapped by the ion-exchange matrix until there are molecules available to exchange with the drug, leaving the drug free to diffuse out of the bead.

In a preferred embodiment, the dispersion media of step (d) is Syrup NF.

In another embodiment, the present invention relates to a method for preparing a liquid form controlled release drug composition, comprising:
(a) allowing the base form of an amine-functional ion-exchange matrix to associate with the acid form of an acid-based drug in the presence of water to form an ion-exchange matrix drug complex, wherein the ion-exchange matrix drug complex is an aqueous gel;
(b) forming beads using the aqueous gel;
(c) coating the beads with a membrane; and
(d) dispersing the beads into a dispersion media.

In certain embodiments, step (a) of the above method further includes the addition of a non-electrolytic soluble component having a molecular weight low enough to diffuse through the membrane. Preferably, the component is lactose.

In certain embodiments, the dispersion media of step (d) further includes the addition of a highly hydrated excipient capable of associating with water such that the water activity outside the beads in the dispersion media will be less than the water activity inside the beads.

In a preferred embodiment, the dispersion media of step (d) is Syrup NF.

It will be understood that when the ion form of a drug is used to prepared the ion-exchange matrix drug complex, the drug is associated with one or more counterions. Illustrative counter-cations include, but are not limited sodium, potassium, and lithium; calcium, magnesium; aluminum and zinc or other similar metals; ammonia and organic amines, such as unsubstituted or hydroxy-substituted mono-, di- or trialkylamines; dicyclohexylamine; tributyl amine; pyridine; N-methyl-N-ethylamine; diethylamine; triethylamine; mono-, bis- or tris-(2-hydroxy-lower alkyl amines), such as mono-, bis- or tris-(2-hydroxyethyl)amine, 2-hydroxy-tert-butylamine or tris-(hydroxymethyl)methylamine, N,N-di-lower alkyl-N-(hydroxy lower alkyl)-amines, such as N,N-dimethyl-N-(2-hydroxyethyl)amine or tri-(2-hydroxyethyl)amine; N-methyl-D-glucamine; and amino acids such as arginine, lysine and the like. Illustrative counter-anions include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, *p*-toluenesulfonate and pamoate (*i.e.,* 1,1'-methylene-bis-(2-hydroxy-3-naphthoate)).

The counterion associated with the ion-form of the drug may be the same or different from the diffusive counterion.

In another embodiment, the present invention relates to a method for preparing a liquid form controlled release drug composition, comprising:
(a) allowing the base form of an amine-functional ion-exchange matrix to associate with the acid form of acid-based drug to form an ion-exchange matrix drug complex; and
(b) dispersing the ion-exchange matrix drug complex into a dispersion media comprising a polyelectrolyte; wherein
   the polyelectrolyte has a negative charge.

Non-limiting examples of useful ion-exchange matrix matrices include those described in Section 5.2 above. In another embodiment, the ion-exchange matrix is formed into a bead using standard forming methods including, but not limited to, granulation, extrusion and/or spheronization.

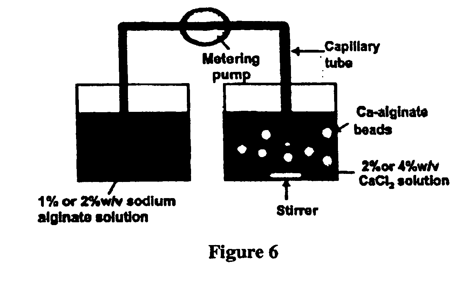

In certain embodiments, the ion-exchange matrix is a hydrophilic colloid formed through methods including, but not limited to coagulation precipitation of a hydrated liquid colloid. In another embodiment, the ion-exchange matrix is a cross-liked hydrophilic colloid. For example, a calcium cross-linked alginate can be formed by adding a 1 or 2 % dispersion of sodium alginate drop-wise into a 2 or 4 % solution of calcium chloride as shown in FIG. 6. After drying, the calcium cross-linked alginate can be coated with a porous diffusion-controlling membrane. The structure of the resultant calcium alginate drug complex is shown in FIG. 3. The alginic backbones 6 are cross-linked by Ca²⁺ cations 7 through -OC(O)-alginic pendant groups 8. Because of the ability of the Ca²⁺ cations 7 to crosslink alginate 6, a solid matrix is formed, which can be formulated into beads. These beads can be "harvested' by filtration, and washed with distilled water to remove excess calcium chloride. The resulting beads have structure because of the cross-linking, and the negative carboxyl groups 9 that are not involved in cross links are neutralized by sodium counterions present in the diffuse double layer 10.

In certain embodiments, the ion-exchange matrix is first activated so that a suitable exchangeable ion is present on the surface of the ion-exchange matrix prior to loading with the electrolytic drug. For a cation-exchange matrix, the activation step typically involves treatment with dilute aqueous base such as, *e.g.*, aqueous NaOH. For an anion-exchange matrix, the activation step involves treatment with dilute acid such as, *e.g.,* aqueous HCl.

In certain embodiments, an ion-exchange matrix is allowed to interact with a drug having a charge opposite that of the ion-exchange matrix, in the presence of water, spontaneously and reversibly forming an aqueous gel, substantially free of spectator ions. The resulting gel is typically added to microcrystalline cellulose and other excipients into a low shear planetary mixer and the resulting wet mass is extruded, spheronized and dried using methods known in the art.

Drug loading may be accomplished by methods known in the art such as, *e.g.,* ion displacement with an electrolytic drug in an aqueous or sufficiently polar non-aqueous dispersion; allowing an acid-functional ion-exchange matrix to associate with the base form of an amine-based drug to form an ion-exchange matrix drug complex; and allowing a free-base-functional ion-exchange matrix to associate with the acid form of a drug to form an ion-exchange matrix drug complex.

By way of illustration, the aforementioned calcium cross-linked alginate beads can be contacted with a concentrated solution of a drug such as propranolol hydrochloride. Because of the concentration gradient, the protonated propranolol cations will diffuse into the beads and displace the sodium counterions. By routine experimentation, one of ordinary skill in the art can determine through specific binding studies the affinity and capacity of the ion-exchange matrix and thereby control the drug loading step. After loading, the calcium cross-linked alginate beads can be harvested by filtration and washed with distilled water to remove unbound propranolol hydrochloride and sodium chloride.

Also by way of illustration, the aforementioned alginic acid can be combined with the base form of a drug such as albuterol in the presence of water. The components will spontaneously and reversibly form a hetergeneous aqueous gel. One of ordinary skill in the art can readily determine the ion-exchange matrix and drug characteristics that allow formation of an aqueous gel substantially free of spectator ions. The resulting gel can be granulated and/or spheronized and/or extruded and the resulting beads dried, and optionally coated. In specific embodiments, excipients such as lactose are incorporated into the beads.

Methods for contacting or loading the ion-exchange matrix with the electrolytic drug include, *e.g.,* bulk mixing a solution phase of the electrolytic drug and the ion-exchange matrix; allowing the ion-exchange material to fall through a column of drug-containing solution; and circulating a solution phase of the electrolytic drug through a bed of the ion-exchange matrix. Changes in drug concentration in the liquid phase can be used to quantitatively monitor the progress of the drug loading.

As noted above, the present invention relates to methods for contacting or loading an acid-functional ion-exchange matrix with the free-base form of an amine-based drug, or a free-base-functional ion-exchange matrix with the acid form of a drug. An advantage of preparing the ion-exchange matrix drug complexes with the non-ionic form of the drugs is that non-aqueous solvents can be employed such as, *e.g.*, methanol, ethanol, propanol, methylene chloride, glycols, and the like, provided that the solvent does not adversely interact with the ion-exchange matrix and the optional porous diffusion-controlling membrane.

Once the contacting step is complete, the solution phase can, optionally, be replenished with additional drug and used again in another contacting step. In another embodiment, the counterions present in the solution phase are removed by contacting the liquid phase with a size selective resin such as, *e.g.*, those used for size exclusion chromatography or size exclusion filtration. The purified liquid phase can then be reused as described above.

As noted above, the ion-exchange matrix drug complexes are dried, and optionally coated with a diffusion-controlling membrane. Drying can be performed using traditional drying equipment known in the art. A fluid bed system is particularly suitable since the beads can be dried and coated in the same unit.

The optional diffusion-controlling membrane is applied to the ion-exchange matrix drug complex in an amount sufficient to control the release of the electrolytic drug from the dispersed phase.

Methods for coating solid form dosages are known in the are *(see* S.C. Porter, Coating of Pharmaceutical Dosage Forms in 57 Remington: The Science and Practice of Pharmacy 894-902 (A.R. Gennaro, ed. 2000, the entire contents of which are incorporated herein by reference). Coating methods useful in the present invention include those using fluidized bed technology including top spray, bottom spray and tangential spray; and pan coating. In one embodiment, the

In one embodiment, the ion-exchange matrix drug complex is spray coated with a spray comprising a diffusion-controlling membrane. In another embodiment, the ion-exchange matrix drug complex is spray coated with a spray comprising a diffusion-controlling membrane and an solvent. Non-limiting examples of solvents useful for spraying diffusion-controlling membrane include water; organic solvents such as, *e.g.*, methanol, ethanol, propanol and dichloromethane; and combinations thereof. The coating may be applied as a solution or suspension.

In another embodiment, the invention relates to a solid phase of the liquid form controlled release drug composition comprising an ion-exchange matrix drug complex as described above; optionally, a diffusion-controlling membrane; and a polyelectrolyte. Such solid can be prepared by, *e.g.*, allowing the liquid component of the dispersion medium of the liquid form controlled release drug composition to evaporate under reduced pressure.

The solid phase of the liquid form controlled release drug composition can be dispersed in a pharmaceutically acceptable liquid prior to use. Non-limiting examples of pharmaceutically acceptable liquids include deionized water; non-aqueous liquids such as, *e.g.*, propylene glycol, glycerin, sorbitol solution and the like, which do not interfere with the intended operation of the invention; and any mixture thereof. In certain preferred embodiments, the liquid further includes a highly hydrated excipient such as sucrose. As such, in a preferred embodiment, the liquid is Syrup NF.

The present invention also relates to methods of forming an ion-exchange matrix drug complex using spray-coating or fluidized bed technology. In one embodiments, the invention relates to a method for forming a multilayer calcium alginate drug precursor, comprising:
(a) providing a seed;
(b) allowing a cation-forming electrolytic drug to be deposited on the seed to form an electrolytic drug layer;
(c) allowing a base to be deposited on the electrolytic drug layer to form a base layer;
(d) allowing alginic acid to be deposited on the base layer to form an alginic acid layer; and
(e) allowing a porous-diffusion controlling membrane to be deposited on the alginic acid layer to form the multilayer calcium alginate drug precursor.

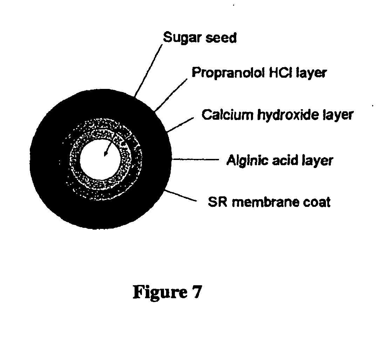

The above-described embodiment is depicted in FIG. 7.

In another embodiment, the method of step (e) further comprises admixing the calcium alginate drug precursor with water that is substantially free of diffusible counterions to form a calcium alginate drug complex.

In another embodiment, the method of step (e) further comprises dispersing the calcium alginate drug complex into a pharmaceutically acceptable liquid to provide a dispersion comprising a pharmaceutically effective concentration of the calcium alginate drug complex. In one embodiment, the dispersion liquid comprises a positively charged polyelectrolyte.

In another embodiment, the method of step (e) further comprises dispersing the calcium alginate drug complex into a pharmaceutically acceptable liquid; and adding a positively charge polyelectrolyte to the dispersion liquid.

In one embodiment, the seed is sugar.

Without being limited by theory, in the above embodiment, Applicants believe that the multilayer calcium alginate drug precursor forms a calcium alginate drug complex by the following process: The multilayer calcium alginate drug precursor, which is typically a dry bead, is immersed in an aqueous environment. Water diffuses into the bead, controlled to some extent by the diffusion-controlling membrane layer. Alginic acid hydrates as water penetrates the diffusion-controlling membrane. As the alginic acid continues to hydrate, water reaches the calcium hydroxide layer, the pH of the "wetted" phases is elevated, and calcium ions begin to diffuse into the alginic acid layer the calcium cross-linking begins to occur. The resultant calcium-alginate gel continues to react with base and cross-link, and water reaches the water-soluble cationic drug. The cationic drug dissolves is in the water, diffuses into the alginic acid layer, and is trapped in the calcium alginate matrix.

An advantage of the above-described embodiment is that all the electrolytic drug remains affixed to the calcium alginate drug precursor.

The concentration of ion-exchange matrix resin drug complex in the liquid form controlled release drug composition can vary over a wide range depending, *e.g.*, on the particular drug, the content of drug in the of ion-exchange matrix resin drug complex; the condition or symptom to be treated; and the age of the patient. In one embodiment, the concentration of ion-exchange matrix resin drug complex in the liquid form controlled release drug composition ranges from about 5% to about 90% by weight based on the total weight of the liquid form controlled release drug composition; in the another embodiment, the weight of ion-exchange matrix resin drug complex ranges from about 10% to about 50% based on the based on the total weight of the liquid form controlled release drug composition; and in the another embodiment, the weight of ion-exchange matrix resin drug complex ranges from about 20% to about 40% based on the based on the total weight of the liquid form controlled release drug composition.

### 5.4 METHODS FOR ADMINISTERING THE LIQUID DOSAGE FORMS

While not restricted in their utility, the liquid sustained release dosage forms of the invention are useful for oral administration to a patient in need thereof.

It is another object of the invention to provide sustained release liquid dosage forms, suitable for once-a-day or twice-a-day administration of highly water soluble electrolytic drugs. In particular, the invention encompasses providing sustained release liquid dosage forms, capable of drug release over the span of about 24 hours, up to 48 hours.

In one embodiment, the invention relates to methods for treating a disease, disorder, condition or symptom, comprising administering a liquid form controlled release drug composition to a patient in need thereof, the drug composition comprising:
(a) a dispersed phase comprising an ion-exchange matrix drug complex comprising a pharmaceutically acceptable ion-exchange matrix and a water-soluble electrolytic drug adsorbed onto the ion-exchange matrix, wherein the surface charge of the ion-exchange matrix is opposite that of the electrolytic drug; and
(b) a dispersion medium comprising a polyelectrolyte having the same charge as the electrolytic drug.

The invention relates to methods for treating a disease, condition or symptom, comprising administering a liquid form controlled release drug composition to a patient in need thereof, the drug composition comprising:
(a) a dispersed phase comprising a bead having an ion-exchange matrix drug complex comprising a pharmaceutically acceptable in-exchange matrix and a electrolytic drug, wherein the surface charge of the ion-exchange matrix is opposite that of the electrolytic drug, and a soluble non electrolytic component;
(b) a diffusion-controlling membrane; and
(c) a dispersion medium comprising a highly hydrated excipient.

In a preferred embodiment, the ion-exchange matrix drug complex comprises alginic acid, albuterol and lactose; the diffusion-controlling membrane is EUGRAGIT®; and dispersion medium is Syrup NF.

Relative to solid oral dosage forms; liquid formulations have the distinct advantages of dosage flexibility and ease of swallowing. In addition, it is possible to administer, in a single volume of liquid, a relatively large quantity of dispersed solid, which would normally require several tablets or capsules.

The dosage forms of the present invention are useful for treating patients who require chronic administration (*e.g.,* patients with dysphagia or cancer patients receiving morphine).

In certain embodiments, the liquid form controlled release drug composition is shaken prior to administration to a patient in need thereof.

In another embodiment, the liquid form controlled release drug composition is further diluted by addition of deionized water, a pharmaceutically acceptable liquid that does not interfere with the intended operation of the drug composition, or any combination thereof.

In another embodiment, the invention relates to a kit comprising the solid phase of the liquid form controlled release drug composition comprising an ion-exchange matrix drug complex as described in any above-described embodiments, optionally having a diffusion-controlling membrane; and a dispersion medium, optionally further comprising polyelectrolyte or a highly hydrated excipient.

In one embodiment, the invention relates to method for treating a condition or symptom, comprising administering a liquid form controlled release drug composition to a patient in need thereof, comprising:
(a) providing a solid phase of the liquid form controlled release drug composition;
(b) dispersing the solid phase into a pharmaceutically acceptable liquid to provide a dispersion comprising a pharmaceutically effective concentration of the solid phase; and
(c) and administering the dispersion to a patient in need thereof.

Without being bound by particular theories, the controlled release composition is administered to a patient, and drug release can be triggered when the suspension is placed in an environment with a relatively high concentration of water and small ions that possess the same charge as the drug. These endogenous ions, which are counterions relative to the surface of the ion-exchange material, diffuse into and swamp the diffuse double layer, reducing its extent or thickness. Endogenous diffusible counterions with positive charge, such as sodium or potassium, or negative charge, such as chloride or phosphate, generally have higher charge density than the drug and are present in greater concentration than the drug. The result is that these ions essentially free the drug from its electrostatic entrapment, allowing it to diffuse out of the aggregated ion-exchange matrix drug complex.

Drug release can also be triggered by the influx of water or gastric fluid when water hydrates the coating or membrane, thereby increasing its porosity, allowing water inside the bead, and facilitating drug dissolution.

The amount of the composition of the invention which will be effective in the treatment, prevention, management or amelioration of one or more symptoms associated with a condition, disease, or disorder can be determined by standard clinical techniques. For example, the dosage of the composition which will be effective in the treatment, prevention, management or amelioration of one or more symptoms can be determined by administering the composition to animal models known to those skilled in the art. In addition, *in vitro* assays may optionally be employed to help identify optimal dosage ranges. Selection of the preferred effective dose can be determined (*e.g.,* via clinical trials) by a skilled artisan based upon the consideration of several factors which will be known to one of ordinary skill in the art. Such factors include the disease to be treated or prevented, the symptoms involved, the patient's body mass, the patient's immune status and other factors known by the skilled artisan to reflect the accuracy of administered pharmaceutical compositions. The precise dose to be employed in the formulation will also depend the seriousness of the symptoms, and should be decided according to the judgment of the practitioner and each patient's circumstances. Effective doses may be extrapolated from dose-response curves derived from *in vitro* or animal model test systems.

In most preferred embodiments, the dosage forms of the invention are capable of controlled release of drug over a span of 8 hours, 12 hours, preferably 18 hours, most preferably 24 hours, up to 48 hours. In such embodiments, patients need only be dosed once daily. The invention encompasses dosage forms that are capable of achieving a linear release profile, preferably with a constant rate of release, most preferably having a zero order release profile.

The following examples are set forth to assist in understanding the invention and should not be construed as specifically limiting the invention described and claimed herein. Such variations of the invention, including the substitution of all equivalents now known or later developed, which would be within the purview of those skilled in the art, and changes in formulations or minor changes in experimental design, fall within the scope of the present invention.

### 6. EXAMPLES

### 6.1 EXAMPLE 1

Example 1 describes a method for making a calcium alginate propranolol hydrochloride ion-exchange matrix drug complex.

A 2% dispersion of sodium alginate in deionized water was prepared and 500 mL was added via a fluid-metering pump at a rate of approximately 1mL.min pumped through a 21 gauge needle to 1000 mL of a stirred solution containing 2% of calcium chloride in deionized distilled water at 25°C as depicted in FIG. 6. The resultant mixture was stirred for about one additional hour at about 25°C. The mixture was filtered and beads washed with 3 X 1750 mL volumes of distilled water to remove excess calcium chloride. The resulting beads have structure because of the cross-linking, and the negative carboxyl groups that are not involved in cross links are neutralized by sodium and/or calcium counterions present in the diffuse double layer.

The dried beads from above were immersed in 2000 mL of 2.5% W/V propranolol hydrochloride solution and stirred for 3 days at about 25°C. The resulting drug-loaded beads were harvested by filtration, and washed with multiple 1250 mL volumes of distilled water until the drug concentration became negligible, (e.g., four washings). The drug-loaded spheres were air-dried at approximately 25°C.

### 6.2 EXAMPLE 2

Example 2 describes the results of equilibrium binding studies involving the exemplary electrolytic drug propranolol hydrochloride and exemplary ion-exchange matrix including sodium alginate, xanthan gum, and gellan gum.

The studies were performed with a two compartment plexiglass dialysis cell (Hollenbeck laboratory) and having a cellulose membrane (molecular weight cutoff of 6000 Daltons) Bel-Art Products (Pequannock, NJ) placed between the two cell compartments. For the sodium alginate studies, one compartment ("the drug compartment") was charged with 15 mL of a 0/97 X 10⁻² molar solution of propranolol hydrochloride in deionized water, while the other compartment ("the polymer compartment") was charged with 15 mL of a 0.0877% W/V solution of the sodium alginate in deionized distilled water. The dialysis cell was shaken at 80 RPM in a thermostatic water bath at 25°C until equilibrium was reached (30 h). The solution was removed from the drug compartment and the concentrations of free drug and polymer-bound drug measured by high performance liquid chromatography ("HPLC").

Results of the binding studies of propanolol hydrochloride using sodium alginate are shown in Table 1.

**Table 1. Binding data for propranolol with sodium alginate.^{a}**

| Total drug concentration (M x 10⁴) | Free drug concentration (M x 10⁴) | Bound drug concentration (M x 10⁴) | Amount bound per gram of polymer (M x 10⁴) | % Bound | (l/g) |
|---|---|---|---|---|---|
| 50.70 | 39.36 | 11.34 | 12.93 | 22.36 | 0.33 |
| 41.20 | 30.35 | 10.84 | 12.37 | 26.33 | 0.41 |
| 30.40 | 20.32 | 10.08 | 11.49 | 33.16 | 0.57 |
| 24.86 | 15.45 | 9.41 | 10.73 | 37.84 | 0.60 |
| 19.88 | 11.28 | 8.60 | 9.81 | 43.28 | 0.87 |
| 14.91 | 7.36 | 7.55 | 8.61 | 50.63 | 1.17 |
| 9.94 | 4.10 | 5.83 | 6.66 | 58.75 | 1.62 |
| 8.95 | 3.57 | 5.37 | 6.12 | 60.02 | 1.71 |
| 7.95 | 2.49 | 5.46 | 6.23 | 68.66 | 2.49 |
| 6.96 | 1.82 | 5.14 | 5.86 | 73.80 | 3.21 |
| 5.96 | 1.43 | 4.53 | 5.16 | 75.94 | 3.60 |
| 5.47 | 1.22 | 4.25 | 4.84 | 77.68 | 3.97 |
| 4.97 | 0.94 | 4.03 | 4.59 | 81.06 | 4.88 |

| | | | | | |
|---|---|---|---|---|---|
| ^{a} The concentration of sodium alginate in the polymer compartment for all studies was 0.877 g/L. | | | | | |

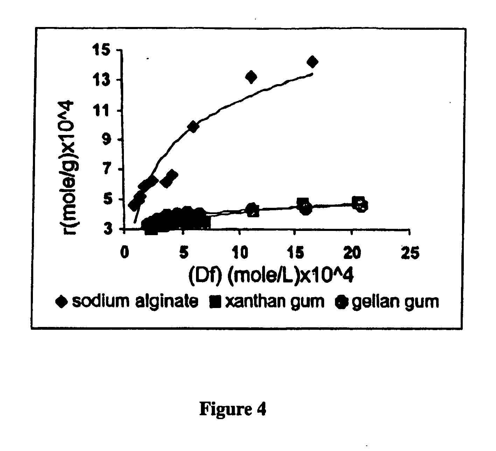

The binding isotherm of propranolol hydrochloride with sodium alginate is shown in FIG. 4 as a plot of r versus D_{f}, where r is the moles of drug bound per gram of polymer and D_{f} is the molar free drug concentration in the system. Fig. 4 also includes data for the cation-exchange matrices gum and gellan gum. The resultant Langmuir-type drug-polymer interaction isotherms indicate that the extent of binding increases with free drug concentration for all three cation-exchange matrices, and the binding is capacity limited.

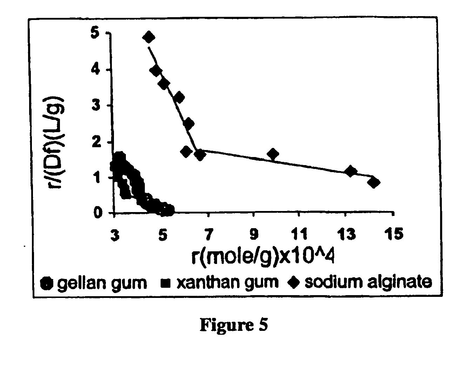

FIG. 5 shows a Scatchard plot of the binding data of r/(D_{f}) versus r for the propranolol hydrochloride with the sodium alginate, xanthan gum or gellan gum. In typical complexation or site specific binding studies with only one type of binding site, the Scatchard plot would be a single straight line where the slope is the association constant (K) and the intercept at the abscissa is equal to the binding capacity (a). FIG. 5 shows that the plots are not single straight lines in the concentration ranges that were studied. Inflection in these plots normally is taken to indicate competitive interactions, the existence of more than one type of binding site, or both. However, in this case nonlinearity is not surprising given that the association is non-specific neutralization in the diffuse double layer.

It does appear, particularly for sodium alginate, that the data could be modeled with two different "binding sites" (*e.g.,* two straight lines). The slopes and the intercepts of the initial portion of each Scatchard plot were determined by linear regression. The residual method was used to determine the second affinity constant (K₂) and binding capacity (n₂) in the high drug concentration range. Using this model, the total drug binding capacity (n_{T}) is then equal to the sum of n₁ and n₂. This model would be consistent with the location of some bound drug in the Stem layer of the DDL and the remainder in the more diffuse region referred to as the Guoy-Chapman layer (these layers are described in Remington: The Science and Practice of Pharmacy, 20th ed., Alfonso Gennaro, Lippincott Williams & Wilkins (2000) at chapter 21: Colloidal Dispersions at page 300; Physical Pharmacy, 4th ed. Alfred Martin, Lea & Febiger (1993) at chapter 15: Colloids, at page 405.) These parameter estimates are provided in Table 2.

**Table 2. Binding capacities and affinity constants for the interaction of propranolol with the cation-exchange matrix matrices sodium alginate, xanthan gum, and gellan gum.**

| Polymer | Binding Capacity, mole/g x 10⁴ | | | Affinity Constant, (M x 10⁴)⁻¹ | |
|---|---|---|---|---|---|
| | 1^{st} Binding Capacity n1 | 2^{nd} Binding Capacity n2 | Total Binding Capacity n_{T} | K₁ | K₂ |
| Sodium Alginate | 7.74 | 7.08 | 14.82 | 1.46 | 0.04 |
| Xanthan Gum | 3.86 | 1.09 | 4.95 | 1.64 | - |
| Gellan Gum | 4.76 | 0.58 | 5.34 | 1.04 | - |

The results in Table 2 show that the first affinity constants (K₁) are similar for different ion-exchange matrices in the low propranolol hydrochloride concentration range. The results also show that sodium alginate has the highest total binding capacities at both high and low propranolol hydrochloride concentration ranges of the three ion-exchange matrices studied.

The results of the binding studies demonstrate the electrostatic association of drug and polymers that are ion-exchange matrices, hence the feasibility of a dosage form comprising ion-exchange matrix drug complexes. The results of the binding studies also provide some basic information for formulating ion-exchange matrix drug complexes. For example, a solution containing a 60 mg (2.02 x 10⁻⁴ mole) dose of propranolol hydrochloride (MW = 295.84) will require an ion-exchange matrix having sufficient capacity to associate with at least 1.82 x 10⁻⁴ moles of the drug in order to meet the arbitrarily selected limit of 10% free drug concentration. For sodium alginate, the maximum binding capacity is 1.48 mmoles per gram (Table 2), so a quantity of about 123 mg of sodium alginate is expected to be sufficient to accommodate the dose of drug under maximum binding conditions. This appears to be a reasonable amount, given the fact that a dosing volume of up to 15 ml would not be problematic (*e.g.,* a colloid concentration of 0.82% W/V).

### 6.3 EXAMPLE 3: COATING THE ION-EXCHANGE MATRIX DRUG COMPLEXES

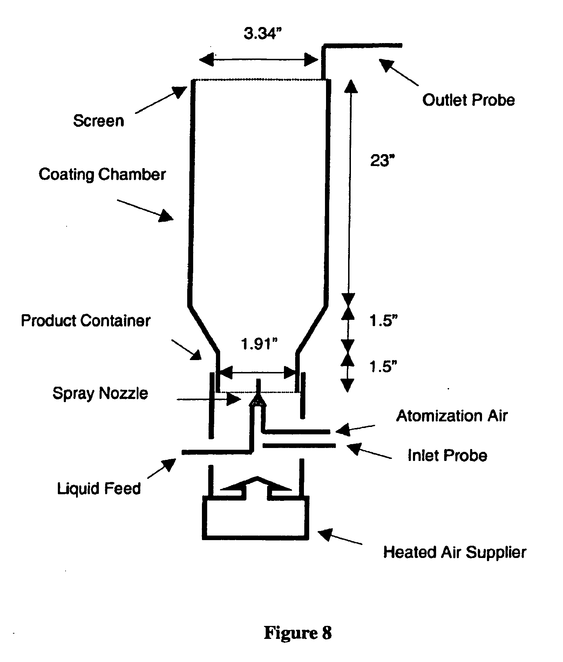

Coating of the ion-exchange matrix drug complexes was performed using the fluid bed coater depicted in FIG. 8, which is useful for processing solids in the 5 to 30 g range. Typically, about 8 g of ion-exchange matrix drug complex was charged to the fluid bed coater. The inlet temperature was set to about 40°C and the bed temperature was set to about 30°C. An aqueous dispersion containing Eudragit®RS 30 D (8.34 g) and triethly citrate (1.67) was prepared, and the dispersion was applied at a spray rate of 0.97ml/min and at an atomization air pressure of about 30 psig. After application was completed, the coated particles were allowed to dry under flowing air in the fluid bed coater. The dried coated particles typically contained about 20-30% by weight of coating based on the total weight of applied coating and ion-exchange matrix drug complex.

### 6.4 EXAMPLE 4: IN VITROTESTING

Example 4 describes the results of an *in vitro* feasibility study showing that both the integrity of the coating and the integrity of the ion-exchange matrix in the dispersed phase (e.g., the beads) is maintained. The salt form of propanolol (propanolol HCl) was employed as a model active ingredient. Propanolol is a weakly basic drug, with a reported pKa of 9.4. At pH values of 7.4 or less, propranolol is protonated and positively charged.

*In vitro* testing was done utilizing the traditional USP Apparatus 2 at 37°C, with a stirring speed of 100 RPM. Ca-alginate drug-loaded spheres coated with Eudragit®RS 30 D and equivalent to 160 mg of propranolol hydrochloride were dispersed using hand shaking in a suspension medium (15 mL) containing a hydrophilic colloid useful both as a polyelectrolyte and suspending agent, and the suspension was added directly to 500 mL of simulated gastric fluid (SGF (*i.e.,* the release medium). After 2 h of agitation, a sufficient quantity of tribasic sodium phosphate was then added to change the pH to 7.5, thus effectively changing the medium to simulated intestinal fluid for the remaining 10 h.

Eight suspensions containing different dispersion mediums were prepared and tested as described above. The hydrophilic colloid polyelectrolytes used in the dispersion media included hydoxypropylmethylcellulose ("HPMC") (F₁ and F₂), xanthan gum (F₃ and F₄), propylene glycol alginate (F₅), chitosan (F₆) and gelatin (F₇). The ionic content, pH; viscosity of the dispersion medium; and percentage of drug released into the dispersion medium were measured and the results provide in Table 3. Ionic content was the sum of the diffusible counter cations K⁺, Na⁺ and Ca²⁺ present in the hydrophilic colloids; pH indicates the extent of drug release from the core, *i.e.,* the lower the pH of the dispersion medium, the greater the extent of drug release; and viscosity is indicative of the effectiveness of the hydrophilic colloid as a suspending agent. The extent of drug release into the suspension medium is also provided in Table 3.

**Table 3. Formulations, properties of the dispersion medium and extent of propanolol hydrochloride release from calcium alginate coated beads dispersed in media containing various hydophilic colloids.**

| **Formulation** | **Components** | **Ionic Content** **(ppm)^{a}** | **pH** | **Viscosity (cP)** | | **Drug Leaching,** **%^{c}** |
|---|---|---|---|---|---|---|
| | | | | **0.6RPM** | **60RPM** | |
| F₁ | 0.8%HPMC | 0^{b} | 6.11 | 1559.7 | 879.8 | 879.8 2.13±0.22 |
| F₂ | 0.8% HPMC Sucrose + Preservatives | 0^{b} | 5.84 | 1559.7 | 947.8 | 0.00 ± 0.00 |
| F₃ | 0.5% Xanthan gum | 13.00 | 5.54 | 3399.3 | 162.0 | 7.95 ± 0.42 |
| F₄ | 0.2% Xanthan gum | 5.20 | 5.00 | 1249.7 | 113.5 | 4.62 ± 0.07 |
| F₅ | 1.95% Propylene Glycol Alginate | 334.63 | 3.34 | 1349.7 | 415.9 | 8.19 ± 0.66 |
| F₆ | 4% Chitosan | 4.79 | 5.57 | | | 4.34 ± 0.13 |
| F₇ | 1% Gelatin | 17.69 | 5.25 | | | 9.02 ± 0.17 |
| F₈ | Deionized water | 0.00 | 6.40 | | | 2.02 ± 0.09 |

| | | | | | | |
|---|---|---|---|---|---|---|
| ^{a}Ion content is the sum of K+, Na+ and Ca2+. ^{b} Actual value from calibration curve is -0.61. ^{c} mean ± SD for 3 replicates, % leaching = free drug content in the medium/ total drug. ^{d} 10% w/v of sucrose was added as a flavoring agent, and the combination 0.2% w/v of methylparaben and 0.0 1 % w/v of propylparaben was added as a preservative. | | | | | | |

The results in Table 3 show that the concentration of diffusible counterions in the dispersion medium strongly influences the extent of release of the drug ion from the ion-exchange matrix drug complex.. The results indicate that additives such as suspending agents must contain a low content of diffusible counterions in order to minimize the extent of release of ionic drug.

The results in Table 3 also show that the strongly anionic polyelectrolyte propylene glycol alginate (F₅) provided a suspension with the highest extent of release of the cationic drug propanolol HCI. This result indicates that additives such as polyelectrolytic suspending agents that have a charge opposite that of the drug ion in the ion-exchange matrix drug complex will affect the levels of free drug concentration in the dispersion medium.

### 6.5 EXAMPLE 5

Example 5 describes a method for making core alginate beads loaded with the drug albuterol.
The formulation for alginic acids beads is depicted in Table 4.

**Alginic Acid Bead Formulation**

| | | | |
|---|---|---|---|
| Albuterol | 5.0% | 12.5 | g |
| Alginic Acid | 5.0% | 12.5 | g |
| Lactose monohydrate (20%) | 20.0% | 50 | g |
| Avicel PH 101 | 70.0% | 175 | g |
| | 100% | 250 | g |

A target drug loading of 5% was used, with equal quantities of albuterol and alginic acid. The albuterol base and the alginic acid powders were placed in a beaker, and 100 mL of water was added. The mixture was agitated using a magnetic stir bar and stirrer. An additional 50 mL of water was added to wash down the sides of the beaker, and agitation was continued until a homogeneous gel was formed.

The microcrystalline cellulose and lactose was placed into the bowl of a low shear mixer and a pre-blend was effected using a spatula. The drug-containing gel was then added while mixing at low speed. The entire quantity of gel was added, to produce a wet mass. The resulting material was extruded using a radial screen attachment with 1 mm screen size. The extrudate was then spheronized at 750 rpm for approximately 30 sec, and then dried overnight in a convection oven at 45°C.

### 6.6 EXAMPLE 6

Example 6 describes a method for making methacrylic acid (Carbopol) core beads loaded with the drug albuterol.

The formulation for the core methacrylic acid (Carbopol) beads is presented in Table 5.

**Table 5. Carbopol Bead Formulation**

| | | | |
|---|---|---|---|
| Albuterol | 2.5% | 6.25 | g |
| Carbopol 974P | 2.5% | 6.25 | g |
| Lactose monohydrate (20%) | 20.0% | 50 | g |
| Avicel PH 101 | 75.0% | 187.5 | g |
| | 100% | 250 | g |

A target drug loading of 2.5% was used with equal quantities of albuterol and alginic acid. Carbopol 974P was employed based on information from the supplier that this grade had greater cross-linking and was designed for oral use.

The microcrystalline cellulose and lactose were placed into the bowl of the low shear mixer and a pre-blend was effected using a spatula. The drug-containing gel was then added while mixing at low speed. The entire quantity of gel was added, plus an additional 10 mL of water, producing a wet mass. The resulting material was extruded using the radial screen attachment with 1 mm screen size. The extrudate was then spheronized at 750 rpm for approximately 30 sec, and then dried overnight in a convection oven at 45 °C.

### 6.7 EXAMPLE 7

Example 7 describes the results of studies involving exemplary coated alginate beads.

The beads were coated in the mini-fluid bed processor with Eudragit RS30D, including triethylcitrate as a plasticizer at a level of 10% of coating solids. The final product was targeted for a coating level of 35%, producing an evenly coated bead with a smooth, glossy surface.

As a crude assessment of physical stability, a 1 g sample of the coated beads was placed in 10 mL of water. Typically, when dispersed in water, the beads will absorb water and rupture within a few hours, producing a paste like substance. Although the dispersion medium turned a little "hazy," these beads maintained their integrity for over 4 days. A sample of the suspended beads was placed on a microscope slide and excess water was removed with an absorbent swab. Observed under a microscope, some water is still present in a pendular state, holding the beads together through capillary forces; however, the beads are clearly intact, and even the shiny surface of the coating is still evident. The alginate-based beads retain their spherical integrity in water and the functional coating appears to be intact also.

Similarly, the Carbopol beads remained intact when exposed to water; however, the behavior was slightly different. Observed over a 75 minute time period, these beads eventually swell in water, and the coating loses its integrity. However, even though the coating loses its integrity, each swollen bead still appears to remain intact. Even after agitation, the discrete character of each bead is retained.

### 6.8 EXAMPLE 8

Example 8 describes the results of studies conducted to evaluate the physical stability and the controlled release of albuterol from coated beads.

The following assay determinations have been done by HPLC using a method based on that in the USP for Albuterol Tablets. Release testing was done using USP Apparatus 2 with paddles operating at 100 RPM. Initially, 750 mL of pH 1.2 buffer was placed in each vessel. After the 1.5 hour time point, 250 mL of pH adjusting solution was added to produce a pH of 6.8 and a volume of 1000 mL. An autosampler was programmed to withdraw approximately 1 mL samples at the following time points: 5, 30, 60, 90 minutes; 4, 8, 12, 16, 20, 24 hrs. The 5 minute point is included to determine the quantity of albuterol that is "immediate" release. This early time point is determines the free drug concentration in the dispersion medium of a suspension dosage form as well as the effectiveness of coating when the sample is dry beads.

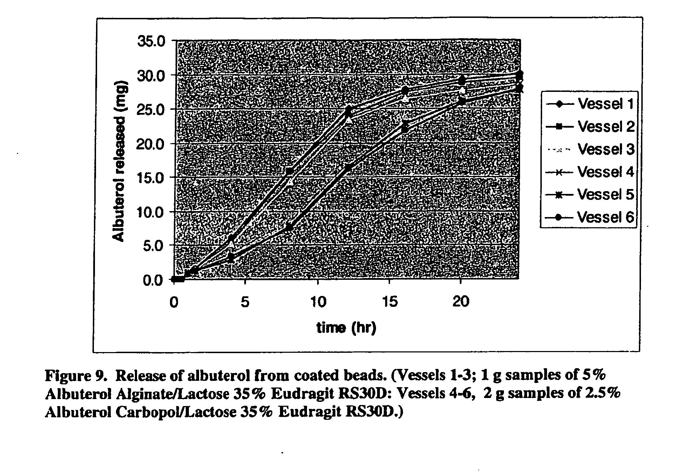

The two products represented in Tables 4 and 5, each of which was coated with 35% Eudragit RS30D, were examined for albuterol release, and the results are presented in Figure 9. Based on the formulation and the target coating weight, each sample tested contains 32.5 mg of albuterol.

Both products demonstrate sustained drug release across a 24 hour period. The carbopol-based formulation presents a profile that is nearly "zero" order, and this product releases drug more slowly than the alginate-based formulation.

When suspensions in Syrup NF (*i.e.,* 65% W/W sucrose) were analyzed, no albuterol peaks were observed on the initial chromatograms, indicating that essentially all the drug remained inside the beads.

Additional samples were prepared for release testing as suspensions containing approximately 50 mg of albuterol in 10 mL liquid. For the alginate-based product, a 1.5 g quantity of beads was used in each system, while a 3.0 g quantity of the less potent carbopol-based beads was used. Three vehicles were used: water, syrup, and a 50:50 mixture ("50% syrup"). The preparations were allowed to stand for 48 hours before testing.

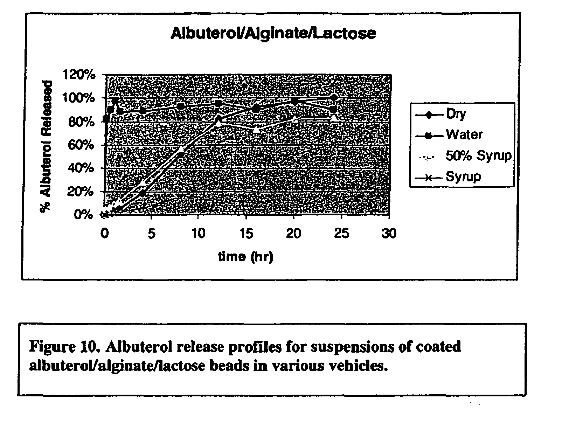

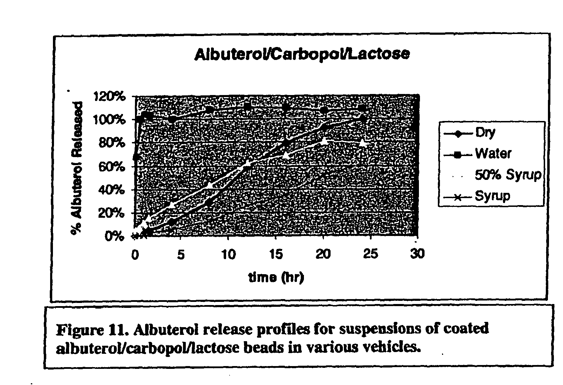

The dissolution results have been translated into % released, with a potency estimate based on the amounts released at 24 hr in Figure 9. The result for each suspension product was then plotted against the appropriate average value obtained for the dry beads in Figure 5. Results for the carbopol-based product are seen in Figure 10, while those for the alginate-based product are in Figure 11.

The behavior of the two formulations is quite similar. In each case, beads suspended in water did not provide any control over albuterol release. Release from the same beads suspended in syrup is slower than that seen for dry beads, while the suspensions in 50% syrup come closer to emulating dry beads.

Based on the results, there is an effect of the syrup vehicle on the permeability of the Eudragit coating. Without being limited by theory, this is most likely due to the high osmotic pressure of syrup itself and commensurate low water activity in the concentrated sucrose solution.

### 6.9 EXAMPLE 9

Example 9 describes the results of studies conducted to evaluate the impact of water activity in the dispersion medium on the performance, physical stability and controlled release of albuterol from coated beads.

The impact of water activity in the vehicle on the performance of products made with lactose-containing beads was examined using the alginate system. Three systems were examined as vehicles, and suspensions were prepared and stored at room temperature for 7 days.

First 50% W/W Sucrose in water was examined. Stability has been demonstrated in the sucrose-containing vehicles, and based on previous results, albuterol release from beads suspended in 40-50% sucrose vehicles did not differ dramatically from what was seen from the coated beads alone. The other two systems examined were methylcellulose dispersions. Results for these products are presented in Figure 8.

In the suspension in 50% W/W sucrose vehicle, compared to the profile for the coated beads, release is somewhat slower. This effect has been seen consistently, without being limited by theory, Applicants believe that additional curing takes place in the hyper-osmotic vehicle. Residual water in the coating would diffuse into the concentrated sucrose solution in the same way that a red blood cell shrinks in an hypertonic environment. Alginate bead formulations of albuterol have been prepared with lactose concentrations of 10, 20, 30, and 40% W/W. The level of lactose has the potential to effect both release rate and stability. For example, in Figure 13, albuterol release is faster for the product with 30% lactose when compared to the one with the 20% level.

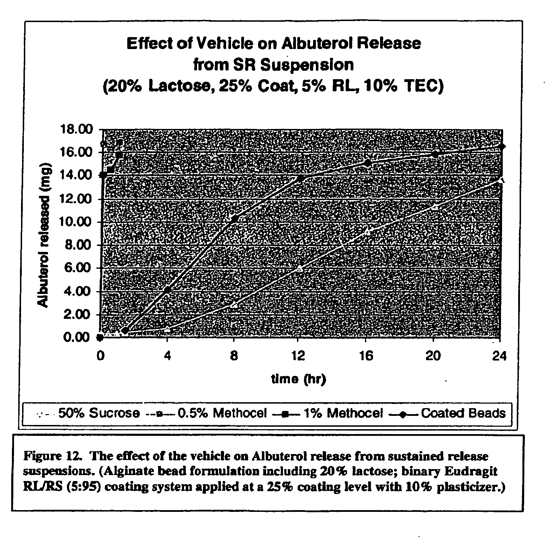

The coating system used for the beads in Figures 12 and 13, is based on a binary combination of Eudragit RL and Eudragit RS; the RL system is more permeable than the RS system. Based on these results, it is clear that the ratio of these polymers can be adjusted to manipulate the characteristics of the internal phase of the suspension.

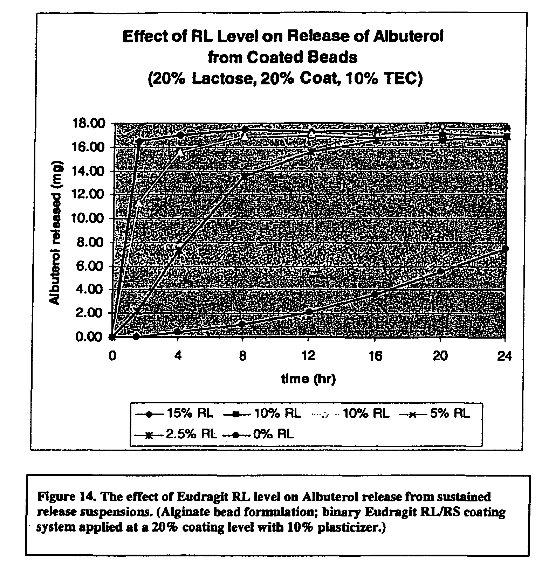

Presented in Figure 14 are release profiles for beads coated with various ratios of the polymers, at the 20% overall coating level.

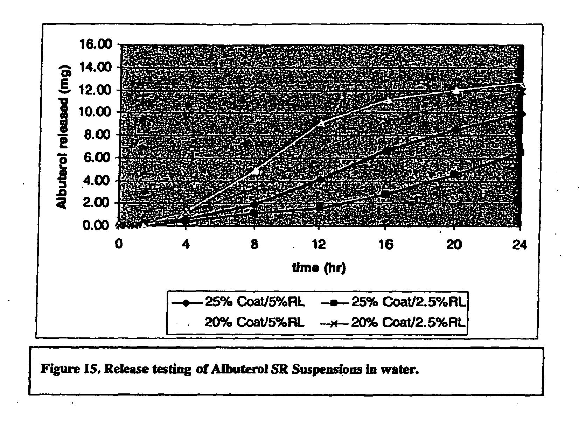

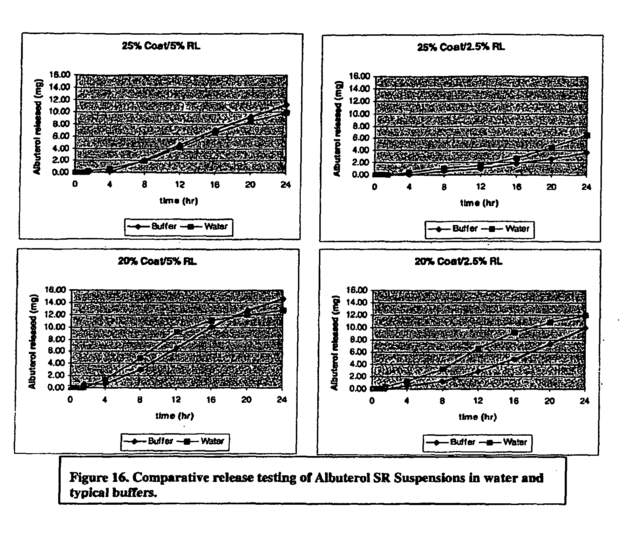

Presented in Figures 15 and 16 are release profiles for beads coated with various ratios of the polymers in water as well as typical buffers.

Design optimization of sustained release suspensions similar to this one, in terms of physical stability and controlled release, requires as a minimum: consideration of the lactose level in the bead formulation; the ratio of permeable (Eudragit RL) to non-permeable (Eudragit RS) polymer in the functional coating system; the plasticizer level; and the concentration of sucrose in the vehicle. Such variations will be apparent to one of skill in the art.

The present invention is not to be limited in scope by the specific embodiments disclosed in the examples which are intended as illustrations of a few aspects of the invention and any embodiments that are functionally equivalent are within the scope of this invention. Indeed, various modifications of the invention in addition to those shown and described herein will become apparent to those skilled in the art and are intended to fall within the scope of the appended claims.

A number of references have been cited, the entire disclosures of which are incorporated herein by reference.

The invention may be described by reference to the following numbered paragraphs :-
1. A liquid form controlled release drug composition, comprising:
   (a) a dispersed phase comprising an ion-exchange matrix drug complex comprising a pharmaceutically acceptable ion-exchange matrix and a water- soluble electrolytic drug associated with the ion-exchange matrix, wherein the surface charge of the ion-exchange matrix is opposite that of the electrolytic drug and a non- electrolytic, soluble, low molecular weight excipient; and
   (b) a dispersion medium.
2. The composition of paragraph 1, wherein the exchange matrix drug complex further comprises a diffusion-controlling membrane coating.
3. The composition of paragraph 2, wherein the diffusion-controlling membrane is selected from the group consisting of ethylcellulose, methylmethacrylate, cellulose esters, cellulose diesters, cellulose triesters, cellulose ethers, cellulose ester-ether, cellulose acylate, cellulose diacylate, cellulose triacylate, cellulose acetate, cellulose diacetate, cellulose triacetate, cellulose acetate propionate, cellulose acetate butyrate, and combinations thereof.
4. The composition of paragraph 3, wherein the diffusion-controlling membrane is ethylcellulose, methylmethacrylate, or combinations thereof.
5. The composition of paragraph 1, wherein the electrolytic drug is albuterol.
6. The composition of paragraph 5, wherein the ion-exchange matrix is alginic acid.
7. The composition of paragraph 1, wherein the ion-exchange matrix includes lactose as said excipient.
8. The composition of paragraph 1, wherein the dispersion medium further comprises a high concentration of a highly hydrated excipient.
9. The composition of paragraph 8, wherein the highly hydrated excipient is sucrose.
10. The composition of paragraph **1,** wherein the dispersion medium comprises Syrup NF.
11. The composition of paragraph 1, wherein the **ion-exchange** matrix drug complex is a particulate or a bead.
12. The composition of paragraph 1, further comprising an excipient selected from the group consisting of sweetening agents, flavoring agents, coloring agents, and any combination thereof.
13. The composition of paragraph 1, further comprising a dispersion additive selected from the group consisting of stabilizing agents, dispersion agents, and any combination thereof.
14. A liquid form controlled release drug composition, comprising:
   (a) a dispersed phase comprising an ion-exchange matrix drug complex comprising a pharmaceutically acceptable ion-exchange matrix and a water- soluble electrolytic drug associated with the ion-exchange matrix, wherein the surface charge of the ion-exchange matrix is opposite that of the electrolytic drug; and
   (b) a dispersion medium comprising a high concentration of highly hydrated excipient.
15. The composition of paragraph 14, wherein the exchange matrix drug complex further comprises a diffusion-controlling membrane coating.
16. The composition of paragraph 15, wherein the diffusion-controlling membrane is selected from the group consisting of ethylcellulose, methylmethacrylate, cellulose esters, cellulose diesters, cellulose triesters, cellulose ethers, cellulose ester- ether, cellulose acylate, cellulose diacylate, cellulose triacylate, cellulose acetate, cellulose diacetate, cellulose triacetate, cellulose acetate propionate, cellulose acetate butyrate, and combinations thereof.
17. The composition of paragraph 16, wherein the diffusion-controlling membrane is ethylcellulose, methylmethacrylate, or combinations thereof.
18. The composition of paragraph 14, wherein the electrolytic drug is albuterol.
19. The composition of paragraph 19, wherein the ion-exchange matrix is alginic acid.
20. The composition of paragraph 14, wherein the ion-exchange matrix further comprises a non-electrolytic, soluble, low molecular weight excipient.
21. The composition of paragraph 20, wherein the low molecular weight excipient is lactose.
22. The composition of paragraph 14, wherein the highly hydrated excipient is sucrose.
23. The composition of paragraph 14, wherein the ion-exchange matrix drug complex is a particulate or a bead.
24. The composition of paragraph 14, further comprising an excipient selected from the group consisting of sweetening agents, flavoring agents, coloring agents, and any combination thereof.
25. The composition of paragraph 14, further comprising a dispersion additive selected from the group consisting of stabilizing agents, dispersion agents, and any combination thereof.
26. A liquid form controlled release drug composition, comprising hydrophilic beads coated with a diffusion controlling membrane comprising albuterol, alginic acid, and lactose, wherein the beads are suspended in a dispersion medium that is 65% sucrose on a weight by weight basis.
27. A method for preparing a liquid form controlled release drug composition, comprising:
   (a) allowing the acid form of an acid- functional ion-exchange matrix to associate with the base form of an amine-based drug to form an ion-exchange matrix drug complex, wherein the ion-exchange matrix drug complex is an aqueous gel;
   (b) forming beads using the aqueous gel; and
   (c) dispersing the beads into a dispersion media comprising a highly hydrated excipient.
28. The method of paragraph 27 wherein the aqueous gel further comprises a non-electrolytic, soluble, low molecular weight excipient.
29. A method for treating a condition or symptom, comprising administering a liquid form controlled release drug composition of paragraph 1 to a patient in need thereof.
30. The method of paragraph 27, wherein the electrolytic drug is albuterol.
31. The method of paragraph 30, wherein the ion-exchange matrix is alginic acid.
32. The method of paragraph 28, wherein the low molecular weight excipient is lactose.
33. The method of paragraph 27, wherein the highly hydrated excipient is sucrose.

## Claims

1. A liquid form controlled release drug composition, wherein said composition is capable of controlled release of the drug over at least 8 hours, comprising:
a. a dispersed phase comprising an ion-exchange matrix drug complex comprising a pharmaceutically acceptable ion-exchange matrix and a water-soluble electrolytic drug associated with the ion-exchange matrix, wherein the surface charge of the ion-exchange matrix is opposite that of the electrolytic drug; and
b. a dispersion medium comprising 50% to 70% on a weight by weight basis of a highly hydrated excipient.

2. The composition of claim 1, wherein the exchange matrix drug complex further comprises a porous diffusion-controlling membrane coating.

3. The composition of claims 2, wherein the diffusion-controlling membrane coating is selected from the group consisting of ethylcellulose, methylmethacrylate, cellulose esters, cellulose diesters, cellulose triesters, cellulose ethers, cellulose ester-ether, cellulose acylate, cellulose diacylate, cellulose triacylate, cellulose acetate, cellulose diacetate, cellulose triacetate, cellulose acetate propionate, cellulose acetate butyrate, and combinations thereof.

4. The composition of anyone of claims 2-3, wherein the diffusion-controlling membrane coating is from about 20% to about 30% by weight based on the total weight of the coating and the ion-exchange matrix drug complex.

5. The composition of anyone of claims 1-4, wherein the electrolytic drug is a cardiovascular drug, respiratory drug, sympathomimetic drug, cholinomemetic drug, adrenergic drug, antimuscarinic drug, antispasmodic drug, skeletal muscle relaxant, diuretic drug, anti-migraine drug, anesthetic, sedative, hypnotic, antiepileptic, psychopharmacologic agent, analgesic, including opioid and non-opioid analgesic, antipyretic, CNS stimulant, antineoplastic, immunosuppressive drug, antimicrobial drug, antihistamine, anti-inflammatory, antibiotic, decongestant, cough suppressant, expectorant or a combination thereof.

6. The composition of anyone of claims 1-5, wherein the electrolytic drug is albuterol.

7. The composition of anyone of claims 1-6, wherein the ion-exchange matrix is a hydrophilic colloid.

8. The composition of anyone of claims 1-7, wherein the ion-exchange matrix is alginic acid.

9. The composition of anyone of claims 1-8, wherein the ion-exchange matrix further comprises a non-electrolytic, soluble, low molecular weight excipient.

10. The composition of claim 9, wherein the low molecular weight excipient is lactose.

11. The composition of anyone of claims 1-10, wherein the highly hydrated excipient is sucrose.

12. The composition of anyone of claims 1-11, wherein the ion-exchange matrix drug complex is a particulate or a bead.

13. The composition of anyone of claims 1-12, further comprising a dispersion additive selected from the group consisting of stabilizing agents, dispersion agents, and any combination thereof.

14. The composition of any one of claims 1-13, wherein the dispersed phase comprises two or more electrolytic drugs.

15. The composition of anyone of claims 1-14, wherein the electrolytic drug of anyone of claims 1-14 is present in its ion form and is associated with one or more counterions.

16. The composition of anyone of claims 1-15, wherein the electrolytic drug is acetophenazine, albuterol , amitriptyline, benztropine, biperiden, bitolerol, bromodiphenhydramine, brompheniramine, buprenorphine, carbinoxamine, chlorcyclizine, chlorpheniramine, chlorphenoxamine, chlorpromazine, clemastine, clonidine, codeine, cyclobenzaprine, cyclizine, cyclobenzaprine, cyproheptadine, desipramine, dexbrompheniramine, dexchlorpheniramine, dextroamphetamine dibucaine, dextromethorphan, dicyclomine, diethylpropion, dihydroengotamine, diltiazem, diphemanil, diphenhydramine, doxepin, doxylamine, ephedrine, ergotamine, fluphenazine, haloperidol, hydrocodone, hydroxychloroquine, hydroxyzine, hyoscyamine, imipramine, isoproterenol, levopropoxyphene, lidocain, maprotiline, meclizine, mepenzolate, meperidine, mephentermine, mesoridazine, metaproterenol, methadone, methdilazine, methscopolamine, methysergide, metoprolol, morphine, nalorphine, nortriptyline, noscapine, nortriptylenepyrilamine, nylindrin, orphenad rine, papaverine, pentazocine, phendimetrazine, phentermine, phenylpropanolamine, phenmetrazine, phenelzine, pibuterol, procaine, prochlorperazine, promazine, propoxyphene, propanolol, protriptyline, pseudoephedrine, pyridostigmine, pyrilamine, quinidine, salmeterol, scopolamine, terbutaline, tetracaine, tranylcypromine, trihexyphenidyl, trimeprazine, tripelennamine, triprolidine, verapamil, or a pharmaceutically acceptable salt thereof.

17. The composition of anyone of claims 1-16, wherein said composition is physically and chemically stable for more than one year.

18. A liquid form controlled release drug composition, wherein said composition is capable of controlled release of the drug over at least 8 hours, comprising hydrophilic beads coated with a diffusion controlling membrane comprising albuterol, alginic acid, and lactose, wherein the beads are suspended in a dispersion medium that is 65% sucrose on a weight by weight basis.

19. A method for preparing a liquid form controlled release drug composition, wherein said composition is capable of controlled release of the drug over at least 8 hours, comprising:
a. allowing the acid form of an acid-functional ion-exchange matrix to associate with the base form of an amine-based drug to form an ion-exchange matrix drug complex, wherein the ion-exchange matrix drug complex is an aqueous gel;
b. forming beads using the aqueous gel; and
c. dispersing the beads into a dispersion media comprising 50% to 70% on a weight by weight basis of a highly hydrated excipient.

20. A method for preparing a liquid form controlled release drug composition, wherein said composition is capable of controlled release of the drug over at least 8 hours, comprising:
a. preparing a dispersed phase by allowing the drug to associate with the ion- exchange matrix to form an ion-exchange matrix drug complex, wherein the surface charge of the ion-exchange matrix is opposite that of the drug; and
b. combining the ion-exchange matrix drug complex with a dispersion medium comprising 50% to 70% on a weight by weight basis of a highly hydrated excipient.

21. The method of anyone of claims 19-20 further comprising coating the ion-exchange matrix drug complex with a porous diffusion-controlling membrane.

22. The method of any one of claims 19-21, wherein the step (a)further comprises addition of a non-electrolytic, soluble, low molecular weight excipient.

23. The method of claim 22, wherein the low molecular weight excipient is lactose.

24. The method of anyone of claims 19-23, wherein the drug is a cardiovascular drug, respiratory drug, sympathomimetic drug, cholinomemetic drug, adrenergic drug, antimuscarinic drug, antispasmodic drug, skeletal muscle relaxant, diuretic drug, anti-migraine drug, anesthetic, sedative, hypnotic, antiepileptic, psychopharmacologic agent, analgesic, including opioid and non-opioid analgesic, antipyretic, CNS stimulant, antineoplastic, immunosuppressive drug, antimicrobial drug, antihistamine, anti-inflammatory, antibiotic, decongestant, cough suppressant, expectorant or a combination thereof.

25. The method of anyone of claims 19-24, wherein the dispersed phase comprises two or more electrolytic drugs.

26. The method of anyone of claims 19-25, wherein the electrolytic drug is acetophenazine, albuterol, amitriptyline, benztropine, biperiden, bitolerol, bromodiphenhydramine, brompheniramine, buprenorphine, carbinoxamine, chlorcyclizine, chlorpheniramine, chlorphenoxamine, chlorpromazine, clemastine, clonidine, codeine, cyclobenzaprine, cyclizine, cyclobenzaprine, cyproheptadine, desipramine, dexbrompheniramine, dexchlorpheniramine, dextroamphetamine dibucaine, dextromethorphan, dicyclomine, diethylpropion, dihydroengotamine, diltiazem, diphemanil, diphenhydramine, doxepin, doxylamine, ephedrine, ergotamine, fluphenazine, haloperidol, hydrocodone, hydroxychloroquine, hydroxyzine, hyoscyamine, imipramine, isoproterenol, levopropoxyphene, lidocain, maprotiline, meclizine, mepenzolate, meperidine, mephentermine, mesoridazine, metaproterenol, methadone, methdilazine, methscopolamine, methysergide, metoprolol, morphine, nalorphine, nortriptyline, noscapine, nortriptylenepyrilamine, nylindrin, orphenadrine, papaverine, pentazocine, phendimetrazine, phentermine, phenylpropanolamine, phenmetrazine, phenelzine, pibuterol, procaine, prochlorperazine, promazine, propoxyphene, propanolol, protriptyline, pseudoephedrine, pyridostigmine, pyrilamine, quinidine, salmeterol, scopolamine, terbutaline, tetracaine, tranylcypromine, trihexyphenidyl, trimeprazine, tripelennamine, triprolidine, verapamil, or a pharmaceutically acceptable salt thereof.

27. The method of anyone of claims 19-26, wherein the ion-exchange matrix is a hydrophilic colloid.

28. The method of claim anyone of claims 19-27, wherein the ion-exchange matrix is alginic acid.

29. The method of anyone of claims 19-28, wherein the highly hydrated excipient is sucrose.

30. Use of the liquid form controlled release drug composition of any one of claims 1-18 for the manufacture of a medicament for treating a condition or symptom in a patient in need thereof.

31. The liquid form controlled release drug composition of anyone of claims 1-18 for use in treating a condition or symptom in a patient in need thereof.

32. The use of claim 30 or the composition of claim 31, wherein the drug comprises a cardiovascular drug, respiratory drug, sympathomimetic drug, cholinomemetic drug, adrenergic drug, antimuscarinic drug, antispasmodic drug, skeletal muscle relaxant, diuretic drug, anti-migraine drug, anesthetic, sedative, hypnotic, antiepileptic, psychopharmacologic agent, analgesic, including opioid and non-opioid analgesic, antipyretic, CNS stimulant, antineoplastic, immunosuppressive drug, antimicrobial drug, antihistamine, anti-inflammatory, antibiotic, decongestant, cough suppressant, expectorant or a combination thereof.
